(19) Europäisches Patentamt
European Patent Office
Office européen des brevets

(11) Publication number: **0 272 779 B1**

(12) **EUROPEAN PATENT SPECIFICATION**

(45) Date of publication of patent specification: **18.05.94**

(51) Int. Cl.[5]: **C12P 21/02**, C07K 13/00, C12N 15/00, A61K 37/02

(21) Application number: **87309409.8**

(22) Date of filing: **23.10.87**

(54) **New forms of colony stimulating factor-1.**

(30) Priority: **24.10.86 US 923067**
**16.04.87 US 39654**
**16.04.87 US 39657**

(43) Date of publication of application:
**29.06.88 Bulletin 88/26**

(45) Publication of the grant of the patent:
**18.05.94 Bulletin 94/20**

(84) Designated Contracting States:
**AT BE CH DE ES FR GB GR IT LI LU NL SE**

(56) References cited:
**WO-A-86/04607**

**The EMBO Journal, vol. 6, no. 9, Sept. 1987, pp. 2693-2698, Heidelberg, DE; M.B. Ladner et al.**

(73) Proprietor: **CETUS ONCOLOGY CORPORATION**
**1400 Fifty-Third Street**
**Emeryville California 94608(US)**

(72) Inventor: **Koths, Kirston Edward**
**2646 Mira Vista Drive**
**El Cerrito, CA 94530(US)**
Inventor: **Halenbeck, Robert Forgan**
**136 Spring Grove Avenue**
**San Rafael, CA 94901(US)**
Inventor: **Kawasaki, Ernest Seigo**
**2601 San Mateo Street**
**Richmond, CA 94804(US)**
Inventor: **Ladner, Martha Ballie**
**4204 Reinhardt Drive**
**Oakland, CA 94619(US)**
Inventor: **Coyne, Mazie Yee**
**4967 Corte Cerritos**
**Pleasanton, CA 94566(US)**
Inventor: **van Arsdell, Janelle Nobel**
**6060 Claremont Avenue, No. E**
**Oakland, CA 94618(US)**
Inventor: **Martin, George Arthur**
**2206 Woolsey Street**
**Berkeley, CA 94705(US)**

(74) Representative: **Bizley, Richard Edward et al**
**HEPWORTH LAWRENCE BRYER & BIZLEY**
**2nd Floor**
**Gate House South**
**West Gate**
**Harlow Essex CM20 1JN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to the use of recombinant technology for production of lymphokines ordinarily produced in low concentration. More specifically, the invention relates to the cloning and expression of new DNA sequences encoding N-terminal deletion muteins of human colony stimulating factor-1 (CSF-1).

The ability of certain factors produced in very low concentration in a variety of tissues to stimulate the growth and development of bone marrow progenitor cells into granulocytes and/or macrophages has been known for nearly 15 years. The presence of such factors in sera, urine samples, and tissue extracts from a number of species is demonstrable using an in vitro assay which measures the stimulation of colony formation by bone marrow cells plated in semisolid culture medium. There is no reliable known in vivo assay. Because these factors induce the formation of such colonies, the factors collectively have been called Colony Stimulating Factors (CSF).

More recently, it has been shown that there are at least four subclasses of human CSF proteins which can be defined according to the types of cells found in the resultant colonies. One subclass, CSF-1 results in colonies containing macrophages predominantly. Other subclasses produce colonies which contain both neutrophilic granulocytes and macrophages; which contain exclusively neutrophilic granulocytes; and which contain neutrophilic and eosinophilic granulocytes and macrophages.

There are murine factors analogous to the first three of the above human CSFs. In addition, a murine factor called IL-3 induces colonies from murine bone marrow cells which contain all these cell types plus megakaryocytes, erythrocytes, and mast cells, in various combinations. Human IL-3 is also known. These CSFs have been reviewed by Dexter, T. M., Nature (1984) 309:746, and Vadas, M. A., et al, J Immunol - (1983) 130:793, Metcalf, D. Science (1985) 229:16-22; Clark, S. C. et al, Science (1987) 236:1229-1237.

The invention herein is concerned with the recombinant production of proteins which are members of the first of these subclasses, CSF-1. This subclass has been further characterized and delineated by specific radioimmunoassays and radioreceptor assays -- e.g., antibodies raised against purified CSF-1 are able to suppress specifically CSF-1 activity, without affecting the biological activities of the other subclasses, and macrophage cell line J774 contains receptors which bind CSF-1 specifically. A description of these assays was published by Das, S. K., et al, Blood (1981) 58:630.

Purification methods for various CSF proteins have been published.

Stanley, E.R., et al, J Biol Chem (1977) 252:4305 reported purification of a CSF protein from murine L929 cells to a specific activity of about 1 x $10^8$ units/mg, which also stimulated mainly macrophage production. Waheed, A., et al, Blood (1982) 60:238, described the purification of mouse L-cell CSF-1 to apparent homogeneity using a rabbit antibody column and reported the first 25 amino acids of the murine sequence (Ben-Avram, C.M., et al, Proc Natl Acad Sci (USA) (1985) 882:4486.

Stanley, E.R., et al, J Biol Chem (1977) 252:4305-4312 disclosed a purification procedure for CSF-1 from human urine and Das, S.K., et al, Blood (1981) 58:630; J Biol Chem (1982) 257:13679 obtained a human urinary CSF-1 at a specific activity of 5 x $10^7$ units/mg which produced only macrophage colonies, and outlined the relationship of glycosylation of the CSF-1 proteins prepared from cultured mouse L-cells and from human urine to their activities. Wang, F.F., et al, J Cell Biochem (1983) 21:263, isolated human urinary CSF-1 to specific activity of $10^8$ U/mg. Waheed, A., et al, disclosed purification of human urinary CSF-1 to a specific activity of 0.7-2.3 x $10^7$ U/mg on a rabbit antibody column (Exp Hemat (1984) 12:434).

Wu, M., et al, J Biol Chem (1979) 254:6226 reported the preparation of a CSF protein from cultured human pancreatic carcinoma (MIAPaCa) cells which resulted in the growth of murine granulocytic and macrophagic colonies. The resulting protein had a specific activity of approximately 7 x $10^7$ units/mg.

Partially purified preparations of various CSFs have also been reported from human and mouse lung-cell conditioned media (Fojo, S.S., et al, Biochemistry (1978) 17:3109; Burgess, A.W., et al, J Biol Chem - (1977) 252:1998); from human T-lymphoblast cells (Lusis, A.J., et al, Blood (1981) 57:13; U.S. Patent, 4,438,032); from human placental conditioned medium to apparent homogeneity and specific activity of 7 x $10^7$ U/mg (Wu, M., et al, Biochemistry (1980) 19:3846).

A significant difficulty in putting CSF proteins in general, and CSF-1 in particular, to any useful function has been their unavailability in distinct and characterizable form in sufficient amounts to make their employment in therapeutic use practical or even possible. The present invention remedies these deficiencies by providing starting material to obtain certain purified muteins of human CSF-1 in useful amounts through recombinant techniques.

(A CSF protein of a different subclass, murine and human GM-CSF has been purified and the cDNAs cloned. This protein was shown to be distinct from other CSFs, e.g., CSF-1, by Gough, et al, Nature (1984) 309:763-767. This GM-CSF protein is further described in WO87/02060, published April 9, 1987, as being useful to treat cancer patients to regenerate leukocytes after traditional cancer treatment, and to reduce the likelihood of viral, bacterial, fungal, and parasitic infection, such as in acquired immune deficiency syndrome (AIDS). Murine IL-3 has been cloned by Fung, M. C., et al, Nature (1984) 307:233. Human and gibbon IL-3 have been cloned by Yang, Y.-C., et al, Cell (1986) 47:3-10, and by Dorssers, L., et al, Gene (1987), in press. See also Yokota, T., et al, PNAS (1984) 81:1070-1074; Wong, G.G., et al, Science (1985) 228:810-815; Lee, F., et al, PNAS (1985) 82:4360-4364; and Cantrell, M.A., et al, PNAS (1985) 82:6250-6254.)

The cloning of a cDNA encoding one form of human CSF-1, specifically the clone designated hereinbelow as pcCSF-17, is also published (Kawasaki, E.S., et al, Science (1985) 230:292-296); PCT application publication No. WO86/04607, published 14 August 1986. Recovery of a clone encoding a "long form" of human CSF-1 is published by Wong, G.G., et al, Science (1987) 235: 1504-1509 and by Ladner, M.D., et al, EMBO J (1987) 6: 2693-2698.

The invention herein relates to previously undisclosed forms of human CSF-1 proteins.

Thus in one aspect, the invention herein relates to a DNA sequence which encodes for a human CSF-1 N∇3 or N∇2 mutein having at least the amino acid sequence of:

(i) N∇3 or N∇2/SCSF/C∇150; or of

(ii) N∇3 or N∇2/LCSF/C∇150; or of

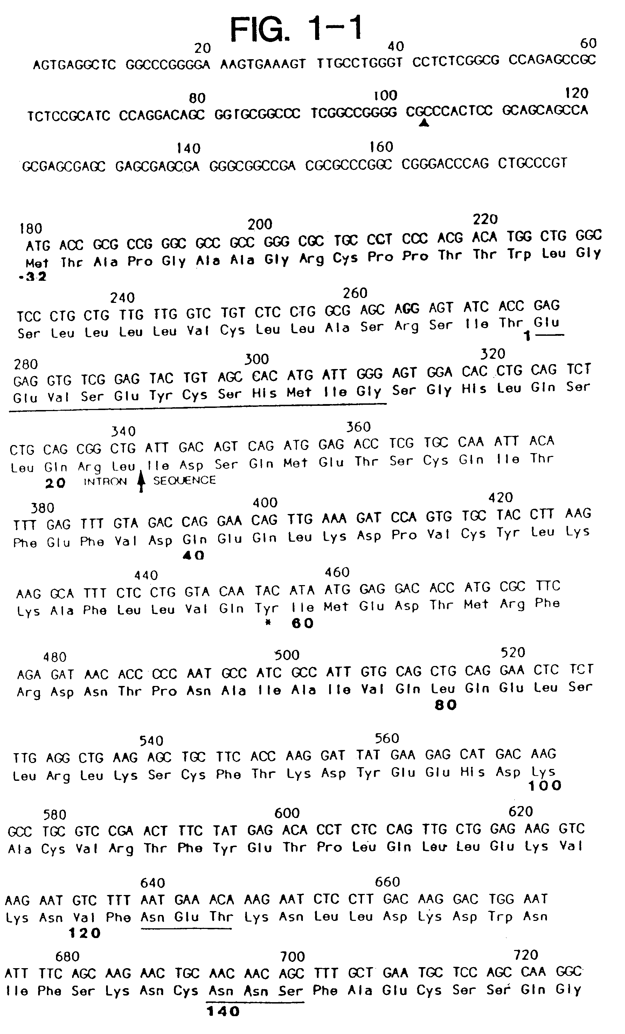

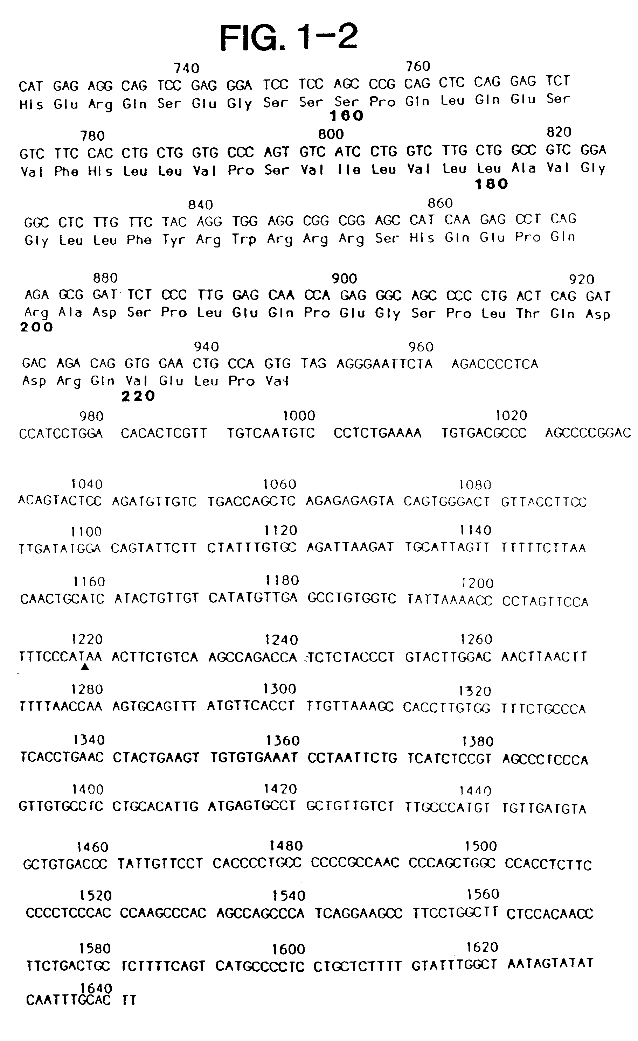

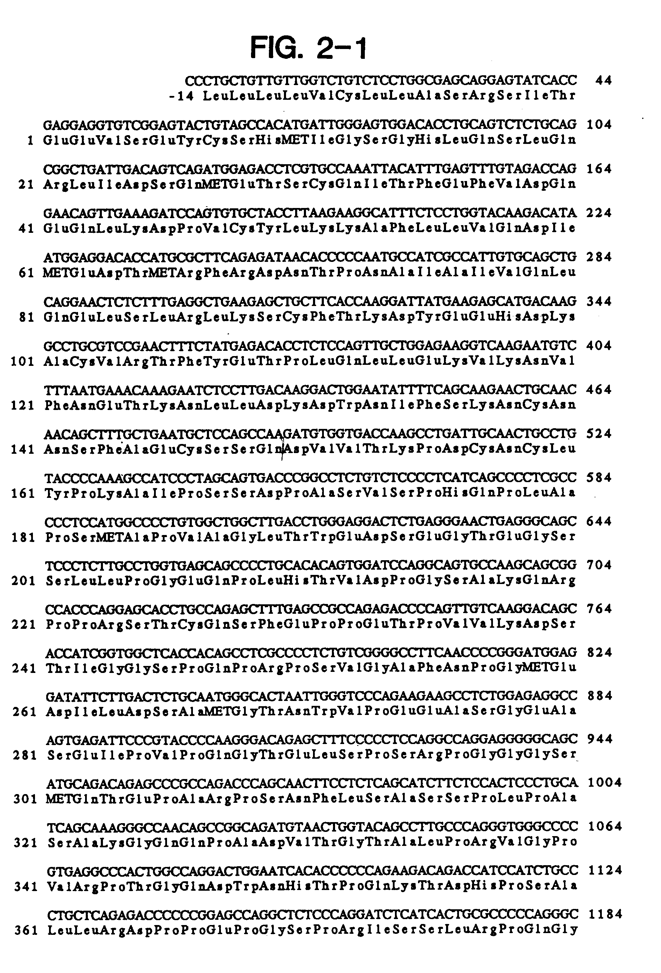

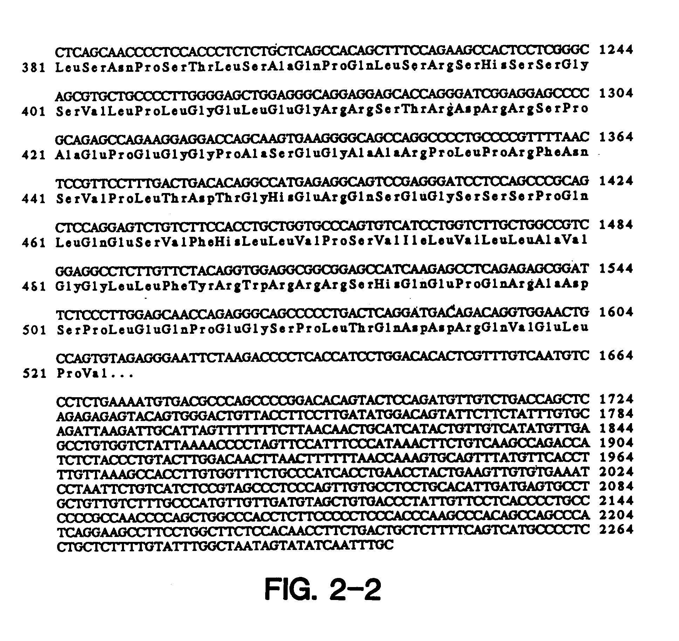

(iii) a modification of either (i) or (ii) in which 1-5 amino acids are substituted or deleted, and wherein dimers of said mutein stimulate the formation of primarily macrophage colonies in the in vitro CSF-1 assay; The human proteins related to the "long" form are described relative to the amino acid sequence shown in Figure 2, designated LCSF, and those related to the "short" form to that shown in Figure 1, designated SCSF.

In other aspects, the invention relates to:-

(a) A vector which comprises a DNA sequence of the invention and a replicon operative in a unicellular organism;

(b) An expression system which comprises a DNA sequence of the invention or a vector of the invention operably linked to suitable control sequences;

(c) Recombinant host cells transformed with an expression system of the invention;

(d) A method of producing a recombinant human CSF-1 polypeptide, which comprises culturing cells as above under conditions effective for the production of said CSF-1.

Figure 1 shows the DNA and deduced amino acid sequences for a cDNA clone encoding a "short form" of human CSF-1 (SCSF), designated pcCSF-17.

Figure 2 shows the cDNA and deduced amino acid sequence for a cDNA encoding a "long form" of huCSF-1 (LSCF).

Figure 3 is a diagram of the genome showing the origin of the short and long forms of human CSF-1.

Figure 4 shows the RP-HPLC of recombinant CSF-1 produced in E. coli from the expression of a gene encoding SCSF/C∇158.

Figure 5 shows RP-HPLC of recombinant CSF-1 produced in E. coli from a gene encoding $asp_{59}$ SCSF/C∇150.

Figure 6 shows RP-HPLC analysis of the expression product of a gene encoding $asp_{59}$ SCSF/N∇3C∇150.

## Modes for Carrying Out the Invention

### A. Definitions

"A protein having colony stimulating factor-1 (CSF-1) activity" refers to a protein which exhibits the spectrum of activity understood in the art for CSF-1 --i.e., when applied to the standard in vitro colony stimulating assay of Metcalf, D., J Cell Physiol (1970) 76:89, it results in the formation of primarily macrophage colonies. Native CSF-1 is a glycosylated dimer; in some instances dimerisation is thought to be necessary for activity. Contemplated within the scope of the invention and within the definition of CSF-1 are both the dimer and monomeric forms. The monomeric form may be converted to the dimer by in vitro provision of suitable conditions, and the monomer is per se useful as an antigen to produce anti-CSF-1 antibodies.

There appears to be some species specificity: Human CSF-1 is operative both on human and murine bone marrow cells; murine CSF-1 does not show activity with human cells. Therefore, "human" CSF-1 should be positive in the specific murine radioreceptor assay of Das, S.K., et al, Blood (1981) 58:630, although there is not necessarily a complete correlation. The biological activity of the protein will generally also be inhibited by neutralizing antiserum to human urinary CSF-1 (Das, S.K., et al, supra). However, in certain special circumstances (such as, for example, where a particular antibody preparation recognizes a CSF-1 epitope not essential for biological function, and which epitope is not present in the particular CSF-1 mutein being tested) this criterion may not be met.

Certain other properties of CSF-1 have been recognized more recently, including the ability of this protein to stimulate the secretion of series E prostaglandins, interleukin-1 and interferon from mature macrophages (Moore, R., et al, Science (1984) 223:178). The mechanism for these latter activities is not at present understood, and for purposes of definition herein, the criterion for fulfillment of the definition resides in the ability to stimulate the formation of monocyte/macrophage colonies using bone marrow cells from the appropriate species as starting materials, under most circumstances (see above) the inhibition of this activity by neutralizing antiserum against purified human urinary CSF-1, and. where appropriate for species type, a positive response to the radioreceptor assay. (It is known that the proliferative effect of CSF-1 is restricted to cells of mononuclear phagocytic lineage (Stanley, E. R., The Lymphokines (1981), Stewart, W. E, II, et al, ed, Humana Press, Clifton, NJ), pp. 102-132) and that receptors for CSF-1 are restricted to these cell lines (Byrne, P. V., et al, Cell Biol (1981) 91:848)) and to placental tropoblast-related cells.

As is the case of all proteins, the precise chemical structure depends on a number of factors. As ionizable amino and carboxyl groups are present in the molecule, a particular protein may be obtained as an acidic or basic salt, or in neutral form All such preparations which retain their activity when placed in suitable environmental conditions are included in the definition. Further, the primary amino acid sequence may, if such can be done without destroying activity, be modified by oxidation or reduction, or augmented by derivatization using sugar moieties (glycosylation) or by other supplementary molecules such as lipids, phosphate, acetyl groups and the like, more commonly by conjugation with saccharides.

The proteins encoded by the genes disclosed herein may also be processed by proteolysis. It is believed that CSF-1 may occur in nature in one or more C-terminally deleted forms. The primary amino acid structure (whether clipped at the C-terminus or not) may also aggregate to form complexes, most frequently dimers. Native human urinary CSF-1 is isolated as a highly glycosylated dimer of 45-90 kd, depending on the method of measurement and identity of the reporter. Native human CSF-1 proteins from other sources, such as monocytes, HL-60, and PanC cell lines have similar characteristics. MIAPaCa-derived CSF-1 seems to be a complex mixture of glycosylated dimeric proteins with monomeric molecular weights of 70, 48, 40, 30 and 26 kd, as determined by immunoblots of SDS-PAGE. Certain aspects of such augmentation are accomplished through posttranslational processing systems of the producing host; other such modification may be introduced in vitro. In any event, such modifications are included in the definition so long as the activity of the protein, as defined above, is not destroyed. It is expected, or course, that such modifications may quantitatively or qualitatively affect the activity, either by enhancing or diminishing the activity of the protein in the various assays. It has been shown by applicants, specifically, that the non-glycosylated monomer may have activity in some assays.

The molecular weight of the deglycosylated monomer has been studied, but definite conclusions could not be drawn in view of the difficulty of assuring lack of proteolysis during fermentation, purification, and in the deglycosylation reaction. Nevertheless, the molecular weight of this deglycosylated dimer was described to be only 14-17 kd by Das, S.K., et al, J Biol Chem (1982) 257:13679-13684. The recombinantly produced CSF-1 reported by Wong, G.G., et al (1987) (supra) appears to have a molecular weight of approximately 21 kd. On the other hand, the molecular weight calculated on the basis of the amino acid sequence deduced for the "short" 224 amino acid form of CSF (SCSF) is on the order of 26 kd, while that of the "long" form (LCSF) is calculated to be on the order of 55 kd. When deleted constructs of these genes are expressed in E. coli (where glycosylation does not occur), they, of course, give rise to proteins of considerably lower molecular weight--17-18 kd for SCSF/C∇150, and about 30 kd for LCSF/C∇221. When LCSF or SCSF are expressed in mammalian or insect cells, higher molecular weights are obtained, but it is difficult to tell to what extent these are due to glycosylation, and to what extent they are due to the primary amino acid sequence per se. As stated above, it appears that the natively produced protein may, in fact, contain C-terminal truncations.

It is, of course, well known that bacterially produced mature proteins which are immediately preceded by an ATG start codon may or may not include the N-terminal methionine in the form as produced and recovered. Accordingly, both forms are included. In addition, slight modification of the N-terminal sequence may aid in the processing of the N-terminal methionine, and it is shown hereinbelow that deletion of residues 1 and 2 (both glutamic acid) or residues 1-3 (glu-glu-val) aids in this manner. Accordingly, these forms are also clearly included in the definition.

In addition to giving expression products which are more efficiently processed at the N-terminus, the proteins produced in E. coli from genes encoding these N∇2 and N∇3 muteins are relatively homogeneous when assessed by RP-HPLC, as compared to the expression products from genes encoding forms which retain the two N-terminal glutamic acid residues. This heterogeneity may be a phenomenon associated with bacterial expression of these proteins since it does not arise when the genes are expressed in mammalian cells, such as CV-1 cells.

As to the effect of N-terminal modifications on N-terminal methionine processing: When constructs encoding the mature protein are expressed intracellularly in E. coli, it appears that essentially none of the protein produced has been processed to remove the N-terminal methionine--i.e., all sequenceable material recovered after protein purification begins with met. However, if the corresponding constructs encoding N∇2-deleted CSF-1 muteins are expressed under similar conditions, 23% of the protein is in N-terminal met-less form. For N∇3 constructs, the percentage of protein which is processed at the N-terminus to remove the methionine increases to approximately 95%.

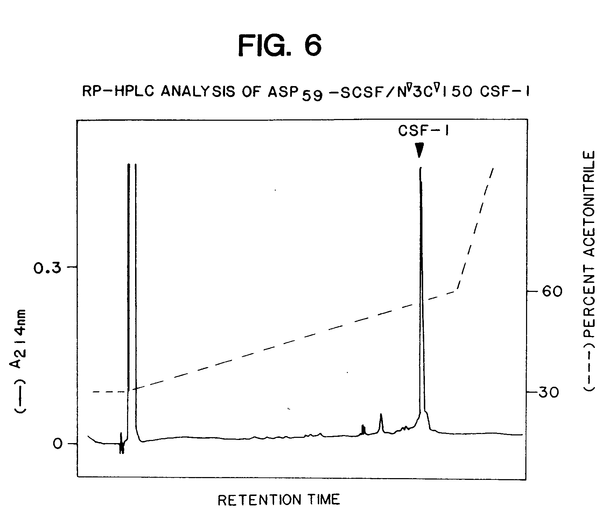

With respect to heterogeneity, when reverse-phase HPLC analysis is performed on the reduced CSF-1 protein produced from various recombinant constructs in E. coli, constructs which encode the mature N-terminus show heterogeneity at two levels. First, there are two peaks of approximately the same molecular weight and the same apparent amino acid composition which bear approximately 70:30 area ratios. There is an additional peak resulting from an internal restart which occurs in clones encoding tyrosine at residue 59, as shown in Figure 6. This complication is removed by redesign of the gene upstream of position 65, as described below. Constructs encoding the N∇2 and N∇3 forms and encoding an asp at residue 59, preferably via a GAT codon, do not show the internal restart fragment and contain only protein having the retention time of the 70% peak of the mature protein compositions.

In summary, in addition to the N-terminal and C-terminal deletions and aggregations, individual amino acid residues in the chain may be modified by oxidation, reduction, or other derivatization, and these proteins may also be cleaved and/or aggregated to obtain fragments which retain activity. Such alterations which do not destroy activity do not remove the protein sequence from the definition, and are specifically included. CSF-1 derived from other species may fit the definition of a protein having activity of "human" CSF-1 by virtue of its display of the requisite pattern of activity as set forth above with regard to human substrate.

Figure 1 shows the amino acid sequence for a particular form of human CSF-1 encoded by the recombinant cDNA clone pcCSF-17. This protein contains 224 amino acids in the mature sequence and a leader sequence of 32 amino acids. As demonstrated herein, the protein produced as the expression product of this clone is ac tive in assays specific for CSF-1, namely, the bone marrow proliferation assay (wherein the activity is destroyed by aaddition of anti-CSF-1 antibodies), colony stimulation assays, and a radioreceptor assay.

The mature protein of the amino acid sequence shown in Figure 1, deduced from the cDNA clone illustrated herein, is designated short CSF-1 (SCSF). Figure 1 shows the presence of a 32 residue putative signal sequence, which is presumably cleaved upon secretion from mammalian cells; SCSF is represented by amino acids 1-224 shown in that figure. Specifically included in the invention are muteins which are monomers and dimers of certain related forms of SCSF that are designated herein by their differences from SCSF.

For convenience, the amino acid sequence of SCSF will be used as a reference and other closely related sequences which have CSF-1 activity will be designated by referring to the sequence shown in Figure 1. Since the N-terminal methionine may or may not be present, both forms are included in all cases wherein the CSF-1 protein is produced in bacteria. The substitution of a particular amino acid will be noted by reference to the amino acid residue which it replaces. Thus, for example, $ser_{90}$SCSF refers to the protein which has the sequence shown in Figure 1 except that the amino acid at position 90 is serine rather than crysteine. Deletions from the termini are noted by N∇ followed by the number of amino acids deleted from the N-terminal sequence, or by C∇ and the position of the last amino acid remaining when residues are deleted from the C-terminal sequence. Thus, "SCSF/N∇4" or the "N∇4 form of SCSF" refers to CSF-1 of Figure 1 wherein the first 4 amino acids from the native N-terminus have been deleted; "SCSF/C∇130" or the "C∇130 form of SCSF" refers to CSF-1 wherein the last 94 amino acids following amino acid 130 have been deleted. Illustrated below are for example, $asp_{59}$SCSF (which contains an aspartic acid residue encoded by the gene at position 59 rather than the tyrosine residue encoded by the cDNA) and SCSF/C∇158 which comprises only amino acids 1-158 of SCSF.

The CSF-1 proteins, which contain amino acid sequences related to those deduced from recovered long form cDNA--i.e., which include a 298 amino acid "extra" segment inserted at residue 150 of the pcCSF-17 encoded protein are considered long forms of the protein, and the amino acid sequence shown as 1-522 in Figure 2 is arbitrarily designated LCSF. Again, this may or may not be preceded by methionine when produced in E. coli. Notation with respect to nuteins of LCSF is analogous to that described with respect to SCSF above.

The 522 amino acid sequence encoded in, for example, pcDBhuCSF-4 and its corresponding clones is that of LCSF and can also be denoted huCSF-1.

The abbreviations used herein also include "huCSF-1" for all human forms of the protein.

As used herein, "discrete peptide" or "mutein" refers to a particular primary amino acid sequence which is not part of a larger sequence. Thus these terms refer to peptide molecules which do not have further N-and C- amino acid sequence extensions. The protein preparations having CSF-1 activity claimed herein may, however, be monomers, dimers or other aggregates of these discrete peptides or muteins.

"Operably linked" refers to juxtaposition such that the normal function of the components can be performed. Thus, a coding sequence "operably linked" to control sequences refers to a configuration wherein the coding sequence can be expressed under the control of these sequences.

"Control sequences" refers to DNA sequences necessary for the expression of an operably linked coding sequence in a particular host organism. The control sequences which are suitable for procaryotes, for example, include a promoter, optionally an operator sequence, a ribosome binding site, and possibly, other as yet poorly understood, sequences. Eucaryotic cells are known to utilize promoters, polyadenylation signals, and enhancers.

"Expression system" refers to DNA sequences containing a desired coding sequence and control sequences in operable linkage, so that hosts transformed with these sequences are capable of producing the encoded proteins. In order to effect transformation, the expression system may be included on a vector; however, the relevant DNA may then also be integrated into the host chromosome.

As used herein "cell", cell line", and "cell culture" are used interchangeably and all such designations include progeny. Thus "transformants" or "transformed cells" includes the primary subject cell and cul tures derived therefrom without regard for the number of transfers. It is also understood that all progeny may not be precisely identical in DNA content, due to deliberate or inadvertent mutations. Mutant progeny which have the same functionality as screened for in the originally transformed cell, are included. Where distinct designations are intended, it will be clear from the context.

"Effective amount" signifies an amount effective to perform the function specified, such as to kill tumors or reduce tumor burden or prevent or cure infectious diseases.

B. Production of CSF-1 for Pharmaceutical Use:

Retrieval of the Gene

Although the existence of a pattern of activity designated CSF-1 had been known for some time, the protein responsible had never been obtained in both sufficient purity and in sufficient amounts to permit sequence determination, nor in sufficient purity and quantity to provide a sueful therapeutic function. Because neither completely pure practical amounts of the protein not its encoding DNA had been available, it had not been possible to optimize modifications to structure by providing muteins, nor had it been possible to utilize this protein in a therapeutic context.

The use of recombinant techniques remedies these defects. As disclosed in PCT W086/04607 (supra), probes based on isolated human urinary CSF-1, N-terminal sequence were used to probe the human genomic library to obtain the full-length gene. The human genomic cloned sequence can be expressed directly using its own control sequences, or in constructions appropriate to mammalian systems capable of processing introns. The genomic sequences were also used as probes for a human cDNA library obtained from a cell line which produces CSF-1 to obtain the pcCSF-17 cDNA encoding protein with CSF-1 activity. This cDNA, when suitably prepared, can be expressed directly in COS or CV-1 cells and can be constructed into vectors suitable for expression in a wide range of hosts. As disclosed in the above application, certain modifications to primary structure also exhibit CSF-1 activity.

As described herein, the human cDNA encoding the 224 amino acid form of the protein was used as a probe to recover the sequences encoding murine CSF-1 from a cDNA bank prepared in λgt10 from L-929 mRNA which had been enriched for CSF-1 production capability. Two clones were recovered which encode a similar 522 amino acid protein. The clones diverge dramatically in the 3' untranslated region. In the longer 4 kb murine clone, the 3' untranslated region is more than 2 kb and bears little resemblance to the corresponding human sequence; the other, shorter 2 kb clone contains approximately 500 bp in the untranslated region and shows considerable homology to the corresponding human DNA.

These long forms of CSF-1 obtained from the murine library were then used as a basis to prepare probes to retrieve the corresponding long human sequence, whose primary structure is encoded in the genome. Based on comparison of the murine cDNAs to the human genomic sequence, a region of the gene which sometimes behaves as an intron region, was seen to encode an amino acid sequence showing considerable homology to the "extra" 298 amino acid segment contained in the murine sequence. This permitted construction of an oligonucleotide probe based on the "extra" DNA which had been, in the murine system, translated to protein. Since the human genomic sequence was available, the probe was designed to accommodate the precise human sequence.

pcCSF-17 had been prepared as a Okayama-Berg vector from MIAPaCa mRNA enriched for CSF-1-encoding materials; however, the cDNA library from which the long form-encoding cDNA was obtained was prepared from total mRNA extracted from MIAPaCa cells and cloned into λgt10. The λgt10 library was first screened using pcCSF-17 sequences as probe, and selected probe-positive candidates were screened using an oligonucleotide probe based on the "extra" translated sequence of the murine cDNA, but modified to correspond to the related region in the human genome. Several clones encoding a corresponding "long form" of a human protein were obtained.

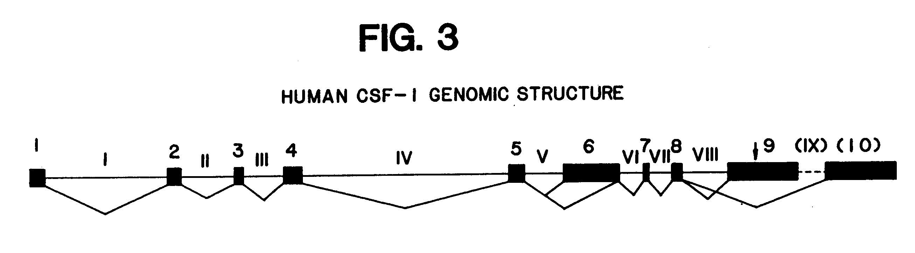

The "long" form apparently arises from a difference in mRNA splicing, as shown in Figure 3. The "extra" coding sequence in the mRNA arises from DNA residing at the upstream end of exon 6; it is spliced out in the short form. Various mRNA-encoded LCSFs also diverge at the 3' end (but not in protein sequence).

The cDNA encoding the "long form" of the protein from the human system can be expressed in manner similar to that discussed for the short form above. Suitable hosts include mammalian cells, so as to obtain more efficient processing of the primary protein product, and, by virtue of ligation into expression vectors, bacteria, yeast, insect cells or other hosts.

Of course, the availability of DNA encoding each of these sequences provides the opportunity to modify the codon sequence so as to generate the mutein forms of this invention having CSF-1 activity.

Thus these tools can provide the complete coding sequence for human CSF-1 from which expression vectors applicable to a variety of host systems can be constructed and the coding sequence expressed. The variety of hosts available along with expression vectors suitable for such hosts permits a choice among posttranslational processing systems, and of environmental factors providing conformational regulation of the protein thus produced.

It is evident from the foregoing that portions of the CSF-1 encoding sequence are useful as probes to retrieve other CSF-1 encoding sequences in a variety of species. Accordingly, portions of the cDNA or genomic DNA encoding at least six amino acids can be replicated in E. coli and the denatured forms used as probes to retrieve additional DNAs encoding CSF-1. Because there may not be a precisely exact match between the nucleotide sequence in the human form and that of the corresponding portion of other species, oligomers containing approximately 18 nucleotides (encoding the 6 amino acid stretch) are probably necessary to obtain hybridization under conditions of sufficient stringency to eliminate false positives. The sequences encoding six amino acids would supply information sufficient for such probes.

C. Suitable Hosts, Control Systems and Methods

In general terms, the production of a recombinant form of CSF-1 typically involves the following:

First a DNA encoding the mature (used here to include all muteins) protein, the preprotein, or a fusion of the CSF-1 protein to an additional sequence which does not destroy its activity or to additional sequence cleavable under controlled conditions (such as treatment with peptidase) to give an active protein, is obtained. If the sequence is uninterrupted by introns it is suitable for expression in any host. If there are introns, expression is obtainable in mammalian or other eucaryotic systems capable of processing them. This sequence should be in excisable and recoverable form. The excised or recovered coding sequence is then preferably placed in operable linkage with suitable control sequences in a replicable expression vector. The vector is used to transform a suitable host and the transformed host cultured under favourable conditions to effect the production of the recombinant CSF-1. Optionally the CSF-1 is isolated from the medium or from the cells; recovery and purification of the protein may not be necessary in some instances, where some impurities may be tolerated,. For example, for in vitro cultivation of cells from which a lymphokine factor will be isolated for administration to a subject, complete purity is sometimes not required. However, direct use in therapy by administration to a subject would, of course, require purification of the CSF-1 produced.

Each of the foregoing steps can be done in a variety of ways. For example, the desired coding sequences can be obtained by preparing suitable cDNA from cellular messenger and manipulating the cDNA to obtain the complete sequence. Alternatively, genomic fragments may be obtained and used directly in appropriate hosts. The constructions for expression vectors operable in a variety of hosts are made using appropriate replicons and control sequences, as set forth below. Suitable restriction sites can, if not normally available, be added to the ends of the coding sequence so as to provide an excisable gene to insert into these vectors.

The control sequences, expression vectors, and transformation methods are dependent on the type of host cell used to express the gene. Generally, procaryotic, yeast, insect or mammalian cells are presently useful as hosts. Since native CSF-1 is secreted as a glycosylated dimer, host systems which are capable of proper posttranslational processing were thought to be preferred. As procaryotic hosts are not capable of effecting glycosylation or controlled dimerization, only if the unglycosylated form can be purified and processed to an active form would these hosts be convenient. This is, as it turns out, the case for CSF-1. CSF-1 can be produced efficiently as a monomer in E. coli and refolded using various techniques to an active, non-glycosylated dimeric form.

However, eucaryotic cells can also be used. In particular, insect or mammalian cells are preferred. If secretion is desired, there is more assurance that the native signal sequence will be recognized by insect or mammalian cell hosts making such secretion possible, and, therefore, purification easier. CSF-1 is stably produced in CHO cells, and can also be produced in stably transformed CV-1 cells and virus-infected insect cells.

In the particular case of human CSF-1, evidence now accumulating indicates that considerable deletion at the C-terminus of the protein may occur under both recombinant and native conditions, and that the in vitro activity of the protein is still retained. It appears that the native proteins isolated may be in some sort of C-terminal truncated form or mixtures thereof, and may exhibit variable C-terminal processing. The activity of these "truncated" forms is clearly established by their deliberate production. The mutein produced from DNA encoding SCSF/C∇150, for example, is fully active in assays for CSF-1, as is that produced from cDNA encoding LCSF/C∇190. The products of recombinant expression of both long and short forms of the genes seem to exhibit subunit molecular weights lower than would be expected from the full length sequence. It is believed that "natural" processing may occur at a variety of proteolytic sites, including, for example in the long form, at the arg residue at 223, the lys residue at 238, the arg residue at 249, or the arg at 411. Since it is clear that certain C terminal shortened forms are active, the constructs used may also include the corresponding shortened forms of the coding sequence.

C.1. Control Sequences And Corresponding Hosts

Procaryotes most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Commonly used procaryotic control sequences which are defined herein to include promotors for transcription initiation, optionally with an operator, along with ribosome binding site sequences, include such commonly used promoters as the beta-lactamase (penicillinase) and lactose (lac) promoter systems (Chang, et al Nature - (1977) 198:1056 and the tryptophan (trp) promoter system (Goeddel, et al Nucleic Acids Res (1980) 8:4057 and the lambda derived $P_L$ promoter and N-gene ribosome binding site (Shimatake, et al, Nature (1981) 292:128), which has been made useful as a portable control cassette. However, any available promoter system compatible with procaryotes can be used.

There was initially some reluctance to utilize bacterial systems in the particular case of CSF-1 in view of its high degree of posttranslational processing, which includes glycosylation and dimerization. In addition, there are a large number of cysteine residues, in particular, in the N-terminal portion of the protein. There are, in fact, a total of ten cysteine residues in the LCSF subunit, the last being at position 225; there are seven in SCSF. Both thus contain cysteine residues at positions 7, 31, 49, 90, 102, 139, 146; the long form has additional cysteines at 157, 159 and 225. It is believed that processing to form dimer includes formation of multiple intrachain and at least one interchain bond. Techniques are available, however, for refolding bacterially produced proteins of this type, and specific protocols which are useful in preparing purified biologically active CSF-1 from bacteria have been developed.

In addition to bacteria, eucaryotic microbes, such as yeast, may also be used as hosts. Laboratory strains of Saccharomyces cerevisiae, Baker's yeast, are most used although a number of other strains are commonly available. While vectors employing the 2 micron origin of replication are illustrated, Broach, J. R., Meth Enz (1983) 101:307, other plasmid vectors suitable for yeast expression are known (see, for example, Stinchcomb, et al, Nature (1979) 282:39, Tschempe, et al, Gene (1980) 10:157 and Clarke, L, et al, Meth Enz (1983) 101:300). Control sequences for yeast vectors include promoters for the synthesis of glycolytic enzymes (Hess, et al, J Adv Enzyme Reg (1968) 7:149; Holland, et al, Biochemistry (1978) 17:4900). Additional promoters known in the art include the promoter for 3-phosphoglycerate kinase (Hitzeman, et al, J Biol Chem (1980) 255:2073), and those for other glycolytic enzymes, such as glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization (Holland, ibid). It is also believed terminator sequences are desirable at the 3' end of the coding sequences. Such terminators are found in the 3' untranslate region following the coding sequences in yeast-derived genes. Many of the vectors illustrated contain control sequences derived from the enolase gene containing plasmid peno46 (Holland, M. J., et al, J Biol Chem (1981) 256:1385) or the LEU2 gene obtained from YEp13 (Broach, J., et al, Gene (1978) 8:121), however any vector containing a yeast compatible promoter, origin of replication and other control sequences is suitable.

It is also, of course, possible to express genes encoding polypeptides in eucaryotic host cell cultures derived from multicellular organisms. See, for example, Tissue Culture, Academic Press, Cruz and Patterson, editors (1973). Useful host cell lines include murine myelomas N51, VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papiloma virus, or avian sarcoma viruses, or immunoglobulin promoters and heat shock promoters. General aspects of mammalian cell host system transformations have been described by Axel; U.S. Patent No. 4,399,216 issued 16 August 1983. It now appears, also that "enhancer" regions are important in optimizing expression; these are, generally, sequences found upstream of the promoter region. Origins of replication may be obtained, if needed, from viral sources. However, integration into the chromasome is a common mechanism for DNA replication in eucaryotes. Plant cells are also now available as hosts, and control sequences compatible with plant cells such as the nopaline synthase promoter and polyadenylation signal sequences (Depicker, A., et al, J Mol Appl Gen (1982) 1:561) are available.

Recently, in addition, expression systems employing insect cells utilizing the control systems provided by baculovirus vectors have been described (Miller, D.W., et al., in Genetic Engineering (1986) Setlow, J.K. et al., eds., Plenum Publishing, Vol. 8, pp. 277-297). These systems are also successful in producing CSF-1.

### C.2. Transformations

Depending on the host cell used, transformation is done using standard techniques appropriate to such cells. The calcium treatment employing calcium chloride, as described by Cohen, S. N., Proc Natl Acad Sci (USA) (1972) 69:2110 is used for procaryotes or other cells which contain substantial cell wall barriers. Infection with Agrobacterium tumefaciens (Shaw, C. H., et al, Gene (1983) 23:315) is used for certain plant cells. For mammalian cells without such cell walls, the calcium phosphate precipitation method of Graham and van der Eb, Virology (1978) 52:546 is preferred. Transformations into yeast are carried out according to the method of Van Solingen, P., et al, J Bact (1977) 130:946 and Hsiao, C. L., et al, Proc Natl Acad Sci (USA) (1979) 76:3829.

### C.3. Probing mRNA by Northern Blot; Probe of cDNA or Genomic Libraries

RNA is fractionated for Norther blot by agarose slab gel electrophoresis under fully denaturing conditions using formaldehyde (Maniatis, T., et al, Molecular Cloning (1982) Cold Spring Harbor Press, pp 202-203) or 10 mM methyl mercury ($CH_3HgOH$) (Bailey, J. M., et al, Anal Biochem (1976) 70:75-85; and Sehgal, P. B., et al, Nature (1980) 288:95-97) as the denaturant. For methyl mercury gels, 1.5% gels are prepared by melting agarose in running buffer (100 mM boric acid, 6 mM sodium borate, 10 mM sodium sulfate, 1 mM EDTA, pH 8.2), cooling to 60°C and adding 1/100 volume of 1 M $CH_3HgOH$. The RNA is dissolved in 0.5 x running buffer and denatured by incubation in 10 mM methyl mercury for 10 min at room temperature. Glycerol (20%) and bromophenol blue (0.05%) are added for loading the samples. Samples are electrophoresed for 500-600 volt-hr with recirculation of the buffer. After electrophoresis, the gel is washed for 40 min in 10 mM 2-mercaptoethanol to detoxify the methyl mercury, and Northern blots prepared by transferring the RNA from the gel to a membrane filter.

cDNA or genomic libraries are screened using the colony or plaque hybridization procedure. Bacterial colonies, or the plaques for phage are lifted onto duplicate nitrocellulose filter papers (S & S type BA-85). The plaques or colonies are lysed and DNA is fixed to the filter by sequential treatment for 5 min with 500 mM NaOH, 1.5 M NaCl. The filters are washed twice for 5 min each time with 5 x standard saline citrate (SSC) and are air dried and baked at 80 °C for 2 hr.

The gels for Northern blot or the duplicate filters for cDNA or genomic screening are prehybridized at 25-42 °C for 6-8 hr with 10 ml per filter of DNA hybridization buffer without probe (0-50% formamide, 5-6 x SSC, pH 7.0, 5x Denhardt's solution (polyvinylpyrrolidine, plus Ficoll and bovine serum albumin; 1 x = 0.02% of each), 20-50 mM sodium phosphate buffer at pH 7.0, 0.2% SDS, 20 μg/ml poly U (when probing cDNA), and 50 μg/ml denatured salmon sperm DNA). The samples are then hybridized by incubation at the appropriate temperature for about 24-36 hours using the hybridization buffer containing kinased probe (for oligomers). Longer cDNA or genomic fragment probes were labeled by nick translation or by primer extension.

The conditions of both prehybridization and hybridization depend on the stringency desired, and vary, for example, with probe length. Typical conditions for relatively long (e.g., more than 30-50 nucleotide) probes employ a temperature of 42 °-55 °C and hybridization buffer containing about 20%-50% formamide. For the lower stringencies needed for oligomeric probes of about 15 nucleotides, lower temperatures of about 25 °-42 °C, and lower formamide concentrations (0%-20%) are employed. For longer probes, the filters may be washed, for example, four times for 30 minutes, each time at 40 °-55 °C with 2 x SSC, 0.2% SDS and 50 mM sodium phosphate buffer at pH 7, then washed twice with 0.2 x SSC and 0.2% SDS, air dried, and are autoradiographed at -70 °C for 2 to 3 days. Washing conditions are somewhat less harsh for shorter probes.

C.4. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 μg of plasmid or DNA sequence is cleaved by one unit of enzyme in about 20 μl of buffer solution; in the examples herein, typically, an excess of restriction enzyme is used to insure complete digestion of the DNA substrate. Incubation times of about one hour to two hours at about 37 °C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacrylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25 °C in 50 mM Tris pH 7.6, 50 mM NaCl, 6 mM $MgCl_2$, 6 mM dTT and 5-10 μM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated. Treatment under appropriate conditions with S1 nuclease results in hydrolysis of any single-stranded portion.

Synthetic oligonucleotides may be prepared by the triester method of Matteucci, et al (J Am Chem Soc (1981) 103:3185-3191) or using automated synthesis methods. Kinasing of single strands prior to annealing or for labeling is achieved using an excess, e.g., approximately 10 units of polynucleotide kinase to 1 nM substrate in the presence of 50 mM Tris, pH 7.6, 10 mM $MgCl_2$, 5 mM dithiothreitol, 1-2 mM ATP. If kinasing is for labeling of probe, the ATP will contain high specific activity $32\gamma P$.

Ligations are performed in 15-30 μl volumes under the following standard conditions and temperatures: 20 mM Tris-Cl pH 7.5, 10 mM $MgCl_2$, 10 mM dTT, 33 μg/ml BSA, 10 mM-50 mM NaCl, and either 40 μM ATP, 0.01-0.02 (Weiss) units T4 DNA ligase at 0 °C (for "sticky end" ligation) or 1 mM ATP, 0.3-0.6 (Weiss) units T4 DNA ligase at 14 °C (for "blunt end" ligation). Intermolecular "sticky end" ligations are usually performed at 33-100 μg/ml total DNA concentrations (5-100 nM total end concentration). Intermolecular blunt end ligations (usually employing a 10-30 fold molar excess of linkers) are performed at 1

μM total ends concentration.

In vector construction employing "vector fragments", the vector fragment is commonly treated with bacterial alkaline phosphate (BAP) in order to remove the 5' phosphate and prevent religation of the vector. BAP digestions are conducted at pH 8 in approximately 150 mM Tris, in the present of $Na^+$ and $Mg^{+2}$ using about 1 unit of BAP per μg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion of the unwanted fragments.

C.5. Modification of DNA Sequences

For portions of vectors derived from cDNA or genomic DNA which require sequence modifications, site specific primer directed mutagenesis is used. This technique is now standard in the art, and is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The plaques are hybridized with kinased synthetic primer at a temperature which permits hybridization of an extract match, but at which the mismatches with the original strand are sufficient to prevent hybridization. Plaques which hybridize with the probe are then picked, cultured, and the DNA recovered. Details of site specific mutation procedures are described below in specific examples.

C.6. Verification of Construction

In the construction set forth below, correct ligations for plasmid construction are confirmed by first transforming E. coli strain MM294, or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., J Bacteriol (1972) 110:667). The isolated DNA is analyzed by restriction and/or sequenced by the dideoxy method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

C.7. Hosts Exemplified

Host strains used in cloning and expression herein are as follows:

For cloning and sequencing, and for expression of construction under control of most bacterial promoters, E. coli strain MM294 obtained from E. coli Genetic Stock Center GCSC #6135, was used as the host. For expression under control of the $P_LN_{RBS}$ promoter, E. coli strain K12 MC1000 lambda lysogen, $N_7N_{53}$cI857 $SusP_{80}$, ATCC 39531 is used.

For M13 phage recombinants, E. coli strains susceptible to phage infection, such as E. coli K12 strain dG98 are employed. The dG98 strain has been deposited with ATCC 13 July 1984 and has accession number 39768.

Mammalian expression has been accomplished herein in COS-7, COS-A2, CV-1, and CHO cells.

D. Preferred Embodiments

The recombinant CSF-1 proteins of this invention can be considered two sets of "basic" proteins, N-terminal muteins of LCSF and N-terminal muteins of SCSF. These proteins have similar but not identical primary amino acid sequences, and various lengths, all of which exhibit, or are specifically cleavable to a mutein which exhibits, the activity pattern characteristic of CSF-1--i.e., they are capable of stimulating bone marrow cells to differentiate into monocytes, predominantly, and, within the limitations set forth in the Definitions section above, are immunoreactive with antibodies raised against native CSF-1 and with the receptors associated with CSF-1 activity. Certain embodiments of muteins referenced to SCSF were not specifically disclosed in PCT application publication No. W086/04607 which is based on the priority of several parent applications hereto. Also not disclosed in said EPO application are the human long forms of CSF-1; specifically LCSF and its related muteins. Certain N- and C-terminal deleted forms of LCSF are especially preferred.

Some specific embodiments of SCSF muteins are disclosed in the EPO application. As set forth in the EPO application, position 59 is variable between asp and tyr--i.e., in addition to the SCSF originally retrieved, included are muteins wherein the SCSF sequence is altered by substitution of asp for tyr at position 59 (i.e., $asp_{59}$ SCSF). Conversely, LCSF has asp at position 59 and can be substituted by tyr ($tyr_{59}$ LCSF).

Disclosed herein is that class of muteins of SCSF and LCSF which lack the N-terminal 2 or 3 residues, and thus has an N-terminal sequence selected from the group consisting of Val-Ser-Glu-Tyr-Cys-Ser and Ser-Glu-Tyr-Cys-Ser.

Also of particular interest are various C-terminal deletion muteins. The EPO application cited above disclosed shortened forms only for SCSF--and only C∇158 and longer. It is not clear from the art to what extent the native proteins are processed from the C-terminus in vivo. It is now established that at least, the C-terminal region back to amino acid 150 of SCSF can be deleted and the protein will retain in vitro CSF-1 activity in the bone marrow proliferation assay. Furthermore, it is believed that the 23 amino acid hydrophobic sequence which extends from position 166-188 in the short form and 464-486 in the long form may have a membrane anchor function and may be separated from the protein when it is transported through the membrane and secreted. This portion may also be responsible for membrane binding per se, and permit CSF-1, when bound to cell membranes to interact with other cells at the site of its binding. These sequences independently may be dispensable in total or in part. In any event, it appears that forms of CSF-1 markedly shorter than either the SCSF or LCSF encoded are found in isolating the native protein, and in the secreted proteins obtained from recombinant production. Present data indicate that expression of constructs encoding SCSF in CV-1 cells may result in SCSF/C∇158 in the supernatant.

Accordingly, included within the invention are CSF-1 proteins which are N∇2 or N∇3 mutein forms of proteins comprising the amino acid sequences containing only the first 150 amino acids of SCSF or LCSF or their interchangeable position 59 tyr or asp muteins as described above (which are therefore identical except for the last amino acid), optionally extended by an arbitrary number of amino acids in further sequence. Among this group, particularly favored are the N∇3 or N∇2 forms of C-terminal deletions corresponding to SCSF/C∇150, SCSF/C∇158, LCSF/C∇150, LCSF/C∇190, LCSF/C∇191, CSF/C∇221, LCSF/C∇223, LCSF/C∇236, LCSF/C∇238, LCSF/C∇249, LCSF/C∇258 and LCSF/C∇411.

Also favored are constructions encoding above-mentioned C-terminal deletions wherein modifications of one or more amino acids preferably 1-5 and more preferably 1-2 amino acids in the sequence from the "basic" sequence have been made.

Thus, the various CSF-1 forms may also contain mutations which do not destroy functionality in regions which are not highly conserved among species, these are 1-5 amino acid deletions and/or substitutions in positions 15-20, 51-52 and/or 75-84. An additional such region is that of 191-193 in SCSF and 489-491 in LCSF. The EPO publication cited above discloses only mutants of SCSF and the C-terminal mutein thereof deleted to C∇158; accordingly, these muteins are new as they relate to both the long form LCSF and its muteins or to the further shortened SCSF/C∇150-157. Specifically, it has been found that $gln_{52}$ forms of both SCSF and LCSF and their muteins are active.

It has also been shown herein that alteration of one or more glycosylation sites can result in active proteins. LCSF has four glycosylation sites--at 122-124, 140-142, 349-351, and at 383-385; SCSF has only the first two. Thus, also included is any mutein containing an inactivating mutation at one or more of these sites.

In addition, the cysteine at position 90 has been shown to be dispensable to immunoreactivity. Accordingly, muteins which are $ala_{90}$ or $ser_{90}$ are included when the purpose intended relies only on antigenicity. The cysteines at 157 and 159 of LCSF are also dispensable for activity and the ser 157, ser 159 and ser 157 ser 159 LCSF muteins (including the N- and C-terminal deleted forms) show improved homogeneity on HPLC. It is also believed that the putative membrane anchor regions of both LCSF and SCSF are dispensable for some types of CSF-1 activity.

Preferred embodiments of CSF-1-Encoding DNA

In addition to modifying the amino acid sequences, the DNA sequence encoding the protein can be modified to assist expression in particular systems. In E. coli, for example, alterations of the codons for the first six amino acids of the native sequence to those favored in bacteria results in higher levels of production for both LCSF and SCSF and for their truncated forms. Thus, vectors (designated O/E below for "over expressing") contain DNAs of the N-terminal coding sequence GAAGAAGTTTCTGAATAT or its N∇ or N∇3 truncations. This is supplied as a synthetic HindIII/BstXI fragment, and differs from the native GAGGAGGTGTCGGAGTAC in the underlined positions shown. One aspect of the invention includes providing the CSF-1-encoding DNA, when it is to be expressed intracellularly in bacteria, in a form wherein an appropriate number of codons at the N-terminus are chosen according to the rules for bacteria-preferred codons.

Also with regard to bacterial expression, the DNA sequences upstream of the ATG at position 65 can be modified to prevent use of this ATG as an internal start site. This is a more serious problem for $tyr_{59}$ than for $asp_{59}$ constructs. A primer of the sequence 5'-3'

*  *  *  *  *

**GGTACAAGATATCATGGAAGATACAATGCGCTTC**

is used in site specific mutagenesis to make the starred alterations in the native gene. No change in the amino acids encoded results.

E. Utility and Administration

The CSF-1 proteins of the invention are capable both of stimulating monocyte-precursor/macrophage cell production from progenitor marrow cells, thus enhancing the effectiveness of the immune system, and of stimulating such funciton of these differentiated cells as the secretion of lymphokines in the mature macrophages. They are also useful anti-infective agents, especially as antiviral and antimicrobial agents.

In one application, these proteins are useful as adjuncts to chemotherapy. It is will understood that chemotherapeutic treatment results in suppression of the immune system. Often, although successful in destroying the tumor cells against which they are directed, chemotherapeutic treatments result in the death of the subject due to this side effect of the chemotoxic agents on the cells of the immune system. Administration of CSF-1 to such patients, because of the ability of CSF-1 to mediate and enhance the growth and differentiation of bone marrow-derived percursors into macrophages, results in a restimulation of the immune system to prevent this side effect, and thus to prevent the propensity of the patient to succumb to secondary infection. Other patients who would be helped by such treatment include those being treated for leukemia through bone marrow transplants; they are often in an immunosuppressed state to prevent rejection. For these patients also, the immunosuppression could be reversed by administration of CSF-1.

In general, any subject suffering from immunosuppression whether due to chemotherapy, bone marrow transplantation, or other, accidental forms of immunosuppression such as disease (e.g., acquired immune deficiency syndrome) would benefit from the availability of CSF-1 for pharmacological use. In addition, subjects could be supplied enhanced amounts of previously differentiated macrophages to supplement thos of the indigenous system, which macrophages are produced by in vitro culture of bone marrow or other suitable preparations treated with CSF-1. These preparations include those of the patient's own blood monocytes, which can be so cultured and returned for local or systemic therapy.

The ability of CSF-1 to stimulate production of lymphokines by macrophages and to enhance their ability to kill target cells also makes CSF-1 directly useful in treatment of neoplasms and infections.

CSF-1 stimulates the production of interferons by murine-derived macrophage (Fleit, H.B., et al, J Cell Physiol (1981) 108:347, and human, partially purified, CSF-1 from MIAPaCa cells stimulates the poly IC-induced production of interferon and TNF from human monocytes. In addition, CSF-1 stimualtes the production of myeloid CSF by human blood monocytes.

Treatment of patients suffering from AIDS with CSF-1, alone or together with erythropoietin and/or an antiviral agent and/or IL-2 is reported in W087/03204, published June 4, 1987. US Patent 4,482,485, issued November 13, 1984, states that CSF-1 can be used in a supporting role in the treatment of cancer. In addition, EP 118,915, published september 19, 1984, reports use of CSF-1 for preventing and treating granulocytopenia and macrophagocytopenia in patients receiving cancer therapy, for preventing infections, and for treating patients with implanted bone marrow. Ralph et al, Cell Immunol (1987) 105:270-279, reports the added tumoricidal effect of a combination of CSF-1 and lymphokine on murine sarcoma TU5 targets.

Thus, in addition to overcoming immunosuppression per se, CSF-1 can be used to destroy the invading organisms or malignant cells indirectly by stimulation of macrophage secretions and activity.

CSF-1 can be employed in conjunction with another lymphokine or cytokine such as, e.g., α-IFN, β-IFN, γ-IFN, IL-1, IL-2, IL-3, IL-4, or TNF to treat tumors.

The CSF-1 of the invention may be formulated in conventional ways standard in the art for the administration of protein substances. Administration by injection is preferred; formulations include solutions or suspensions, emulsions, or solid composition for reconstitution into injectables. Suitable excipients include, for example, Ringer's solution, Hank's solution, water, saline, glycerol, dextrose solutions, and the like. In addition, the CSF-1 of the invention may be preincubated with preparations of cells in order to stimulate appropriate responses, and either the entire preparation or the supernatant therefrom introduced into the subject. As shown hereinbelow, the materials produced in response to CSF-1 stimulation by various types of blood cells are effective against desired targets, and the properties of these blood cells themselves to attack invading viruses or neoplasms may be enhanced. The subject's own cells may be withdrawn and used in this way, or, for example, monocytes or lymphocytes from another compatible individual employed in the incubation.

It is preferred that the present "human" forms of CSF-1 be used in pharmaceutical compositions.

F. Cloning and Expression of Human CSF-1

The following illustrates the methods used in obtaining the coding sequence for human CSF-1, for disposing this sequence in expression vectors, and for obtaining expression of the desired protein. Of course, retrieval of the DNA need not be repeated; the disclosed sequences may be obtained partially or totally by synthesis.

F.1. Purification of Native Human CSF-1 and Probe Design

Human urinary CSF-1 was partially purified by standard methods as described by Das, S. K., et al, Blood (1981) 58:630, followed by an affinity purification step using a rat monoclonal antibody to murine CSF-1, designated YYG106, attached to a Sepharose 4 B column (Stanley, E.R., Methods Enzymol (1985) 116:564). The final step in purification was reverse phase HPLC in a 0.1% TFA/30% acetonitrile - 0.1% TFA/60% acetonitrile buffer system. The details and results of purification and design of probes are described in PCT application W086/04607 (supra).

F.2. Preparation of the Human Genomic Sequence

A human genomic sequence encoding CSF-1 was obtained from the Maniatis human genomic library in λ phage Charon 4 using probes designed to encode the N-terminal sequence of human protein, as described in PCT application W086/04607 (supra). A Charon 4A phage containing the CSF-1 sequence as judged by hybridization to probe as described below, and designated phCSF-1, was deposited with the American Type Culture Collection (ATCC) on 2 April, 1985 and has accession no. 40177. Upon later study of this phage, it was found that rearrangements and/or deletions had occurred and the correct sequences were not maintained. Therefore, an alternative colony obtained from the genomic library in identical fashion, and propagated to confirm stability through replication, was designated phCSF-1a and was deposited with ATCC on 21 May 1985, and given accession number 40185. phCSF-1a contained an 18 kb insert and was capable of generating restriction enzyme digests which also hybridized to probe, and it was used for sequence determination and additional probe construction.

The entire 18 kb gene contained in phCSF-1a was sequenced. The gene contains 9 exons separated by 8 introns when compared to the pcCSF-17 sequences of Figure 1. The regions of the mature protein cDNA correspond exactly to the genomic exon codons except for codon 59; the "long form" of the human CSF-1 protein shown as extended coding region at the 5' end of exon 6, as further described below.

Figure 3 represents a schematic of the genomic structure for human CSF-1. The first exon contains untranslated 5' sequence and the first 13 amino acid residues of the signal sequence. The remaining 19 amino acids of the signal sequence and first 22 amino acids of the mature CSF protein are encoded in the second exon. The stop codon appears in exon 8, which also includes 9 bp of the 3' untranslated sequence. The gene spans approximately 18 kb and contains at least 9 exons, most likely 10, ranging in size from 63 bp to a presumed maximum of 2-3 kb. It is believed that the 3' untranslated sequence can be completed either with exon 9 or exon 10. Although two separate exons are shown, it is not clear whether there is a 9th intron and 10th exon or whether an alternative splice site is within exon 9. As will be described further below, the short form of CSF-1 results from the utilization of the splice site toward the 3' end of exon 6; the long form results from the splice site at what is shown in Figure 3 as the beginning of that exon.

The sizes of the exons and introns illustrated in Figure 3 are as follows:

| EXON | SIZE (bp) | INTRON | SIZE (kb) |
|---|---|---|---|
| 1 | 217 | I | 3.1 |
| 2 | 123 | II | 1.3 |
| 3 | 63 | III | 1.3 |
| 4 | 171 | IV | 4.6 |
| 5 | 148 | V | 1.2 or 2.1 |
| 6 | 1025 or 131 | VI | 0.6 |
| 7 | 49 | VII | 0.3 |
| 8 | 60 | VIII | 0.8 |
| 9 | 1400 (?) | (IX) | ? |
| (10) | ? | | |

That the genomic clone is transcribed into a number of RNA transcripts depending on the splice sites is verified by Northern analysis. MIAPaCa cells were induced under serum free conditions with 50 ng/ml PMA and 10 $\mu$M retinoic acid for 3 days in three successive inductions and mRNA was isolated after the third induction according to the general procedure described below. The mRNA isolate was electrophoresed on a 1% agarose gel containing 2.5 M formaldehyde, blotted onto nitrocellulose and probed with the appropriate oligonucleotide. A number of bands, corresponding to mRNAs of 16S, 18S, 26S, 28S, and a pool at 22-25S are obtained.

MLO6, a 20-mer which corresponds to the coding sequence in exon 8 of the genome, hybridizes to the transcripts of all of these; thus verifying that all encode CSF-1. The sequences of exon 8 are, of course, common to both long and short forms.

GM11, a 40-mer which resides at the 5' end of long exon 6, and thus corresponds to the "extra" insert containing 894 bp in the long form sequence, hybridizes to the transcripts at 28S and the cluster at 22-25S, but not to the remaining three. Thus, apparently, these are transcripts corresponding to the long form.

A 20-mer, JV30, which matches the 5' end of exon 9 of the genome, hybridizes to the 22-25S cluster and to the 16S and 18S mRNAs, but not to the 26S or 28S. Therefore, 26S and 28S appear to result from the processing shown to include putative exon 10 in Figure 3. An additional probe, a 4.2 kb probe made from a human CSF-1 genomic fragment which begins 1500 bp into exon 9, hybridizes only to the 26S and 28S transcripts.

The genomic fragment encoding CSF-1 can be used for expression in eucaryotic cells capable of processing introns. In order to express the genomic fragment, the insert is ligated into a suitable host vector such as a bovine papilloma virus or other mammalian virus vector immediately downstream from a suitable promoter such as the murine or human metallothionein promoter.

F.3. Retrieval of cDNA Encoding Human CSF-1 pcCSF-17 (SCSF)

The human derived pancreatic carcinoma cell line MIAPaCa-2 was used as a source of mRNA to validate probes and for the formation of a cDNA library containing an intronless form of the human CSF-1 coding sequence. The MIAPaCa cell line produces CSF-1 at a level approximately 10-fold below that of the murine L-929 cells. pcCSF-17 was obtained from this library, as described in PCT application W086/04607.

Briefly, a 300,000 clone library obtained from an enriched MIAPaCa mRNA by the Okayama and Berg method was then probed under conditions of high stringency, using the exon II probe derived from the genomic DNA. Ten colonies hybridizing to the probe were picked and colony purified. These clones were assayed for the presence of CSF-1 encoding sequences by transient expression in COS-7 cells. The cloning vector, which contains the SV40 promoter was used per se in the transformation of COS-7 cells.

Plasmid DNA was purified from 10 positive clones using a CsCl gradient, and COS-7 cells were transfected using a modification (Wang, A.M., et al, Science (1985) 228:149) of the calcium phosphate coprecipitation technique. After incubation for three days, CSF-1 production was assayed by subjecting the culture supernatants to the radioreceptor assay performed substantially as disclosed by Das, S.K., et al. Blood (1981) 58:630, and to a colony stimulation (bone marrow proliferation) assay performed substantially as disclosed by Prystowsky, M.B., et al, Am J Pathol (1984) 114: 149. Nine of the ten clones picked failed to show transient CSF-1 production in COS-7 cells. One clone, which did show expression, was cultured, the plasmid DNA was isolated, and the insert was sequenced. The DNA sequence, along with the deduced amino acid sequence, is shown in Fgiure 1. The full length cDNA is 1.64 kb and encodes a mature CSF-1 protein of 224 amino acids (SCSF), as shown in Figure 1. The clone was designated CSF-17 with Cetus depository number CMCC 2347 and was deposited with the American Type Culture Collection on 14 June 1985, as accession no. 53149. The plasmid bearing the CSF-1 encoding DNA was designed pcCSF-17.

Clones Encoding LCSF

pcCSF-17, prepared as described above, was used as a probe to obtain additional CSF-1 encoding clones from a human cDNA library. Total mRNA was prepared from MIAPaCa cells exactly as described in PCT application W086/04607 (supra) and used to obtain a cDNA library in λgt10 phage vectors by ligating the reverse transcripts to EcoRI linkers and inserting the EcorRI digest of the cDNA thus provided into the EcoRI site of λgt10, as described by Huynh, T.V., et al, in DNA Cloning Techniques: A Practical Approach, IRL Press, Oxford, 1984, D. Glover, ed.

Over one million phage were screened using a single-stranded highly labeled probe derived from CSF-17 using standard procedures, which are briefly summarized as follows.

An EcoRI fragment of pcCSF-17 DNA, which includes the entire coding sequence for CSF-1 (there is an EcoRI site immediately following the stop codon of the coding sequence in pcCSF-17) was inserted into M13, and a labeled second strand synthesized as follows: Approximately 1 pmol of M13 containing the single-stranded EcoRI-digested pcCSF-17 was annealed with 10 pmol of sequencing primer in 20 mM Tris, pH 7.9, 20 mM $MgCl_2$, 100 mM NaCl, and 20 mM β-mercaptoethanol at 67°C for 5 min and then transferred to 42°C for 45 min. The annealed preparation was supplied with 100 µmol each of dATP, dCTP, and dTTP, and 2.5 µmol $P^{32}$-labeled (α)dGTP, along with 5 U Klenow fragment. The reaction was incubated at 37°C for 20 min, and the DNA recovered on a P-6 dG (Bio-Rad) spin column and boiled for 10 min to separate the strands.

The probes, thus prepared, were used to screen the plaques (which had been transferred to nitrocellulose) by hybridization under stringent conditions (50% formamide, 5x SSC, 5x Denhardt's) at 42°C for 18 hr.

Approximately 150 phage plaques were positive. Five of these 150 probe-positive plaques were further probe positive using the oligonucleotide JD11, a 14-mer that is complementary to the bases in the region of the exon 2 and 3 splice junction, when hybridized at 45°C overnight in 5x SSC, 5x Denhardt's.

The 5 JD11 positive clones were then subjected to hybridization to the oligonucleotide GM11, which has the sequence complementary nucleotides 506-545 in Figure 2. As described above, this sequence is an exact match to that portion of the human genomic sequence which corresponds to the "extra" portion of the murine cDNA, described below, that encodes the "extra" 298 amino acid segment in the "long forms" of the murine CSF-1 protein. Hybridization was in 20% formamide, 5x SSC, 5x Denhardt's at 45°C for 18 hr. Three clones were positive, including the relevant clones 4 and 25.

The complete DNA sequence for the pertinent coding regions of the cDNA inserts in clones 4 and 25, along with the deduced amino acid sequence, are shown in Figure 2. The sequence was derived by integrating the known sequence of the genomic clone, phCSF-1a, described above, using the 298 amino acid "extra" insert of the murine sequences described below as a guide to deduce the complete sequence shown. The sequence depicted shows the splicing of the "extra" segment, sufficient to encode 298 "extra" amino acids contained in the gene at the 5' side of exon 6, in to the sequence of pcCSF-17 before the first nucleotide of the codon for the Gly residue at amino acid position 150 into reading frame with the remaining CSF-17 sequence. The insert changes the codon at 150 to a codon for aspartic acid, the subsequent codon at the end of the insert is reconstituted to a Gly, and the remaining sequence of residues containing with His-Glu-Arg etc. down to the C-terminal Val residue remain the same as in pcCSF-17. LCSF is otherwise identical in sequence to SCSF up to residue 150, except that it has Asp rather than Tyr at position 59.

Two of the clones, 4 and 25, were cloned into M13mp18 or M13mp19 for sequencing to confirm the results shown in Figure 2 using the EcoRI restriction sites. These two clones appeared identical from restriction enzyme analysis. The complete sequence of clone 4 was determined and is that shown in Figure 2; partial sequencing of clone 25 gives consistent results. They were then subcloned into the modified Okayama-Berg vector pcDB, which was prepared from the published Okayama-Berg vectors pcDV1 and pL1 (Okayama, H., et al, Mol Cel Biol (1983) 3:280-289) as follows:

pcDV1 was digested with HindIII and KpnI and the large fragment, providing $Amp^R$, the pBR322 origin or replication, and a polyadenylation site, was isolated. pL1 was digested with HindIII and PstI and the small fragment, providing the SV40 promoter and origin of replication, was isolated. These fragments were religated in a three-way ligation with the synthetic, KpnI/PstI-digested oligonucleotide fragment

CTGCAGGAGCTCAGATCTTCTAGAGAATTCTCGAGCGGCCGCATCGATGGTACC
GACGTCCTCGAGTCTAGAAGATCTCTTAAGAGCTCGCCGGCGTAGCTACCATGG

to obtain pcDB, a plasmid corresponding to pcD-x shown in the reference, wherein "x" is replaced by a polylinker. Thus, pcDB contains the SV40 early promoter and an immediately downstream linker followed by a polyadenylation site. Sequences ligated into the polylinker region should be expressed under the control of the SV40 early promoter.

Before testing expression, because clones 4 and 25 appeared to be missing some 5' end sequences as compared to CSF-17, the upstream portions from pcCSF-17 were substituted for those of clones 4 and 25.

The protocol for this substitution was as follows: pcCSF-asp59-BamBcl (see below) was digested with SmaI, which cuts at the extreme 5' end of the untranslated sequence (see Figure 1) and the linearized plasmid was ligated to EcoRI linkers and resealed. The religated plasmid was then digested with EcoRI, which removes the entire coding region from the SmaI site at the extreme 5' end to the EcoRI site immediately downstream of the stop codon. This was ligated into the polylinker of pcDB at the EcoRI site. The coding sequences downstream of the BstXI site, which site is located at approximately the codon for amino acid 8 of the CSF-17 mature protein sequence (see Figure 1) was removed by digestion was BstXI and KpnI. (KpnI cuts into the linker slightly downstream of the EcoRI site past the stop codon.) This deleted downstream sequence was replaced by the analogous BstI/KpnI fragment from pM13-4 and pM13-25. The resulting clones, pcDBhuCSF-4 and pcDBhuCSF-25 thus contained the coding sequences downstream of the codon for approximately amino acid 8 from clones 4 and 25, respectively, and the upstream sequences from pCSF-17. The ligated sequences are in reading frame and under control of the SV40 early promoter.

Mutein-Encoding Sequences/Eucaryotic Expression Vectors

Modifications were made of the pcCSF-17 inserts to provide corresponding plasmids encoding muteins of the SCSF protein. For site-specific mutagenesis, pcCSF-17 and M13mp18 were digested with the same restriction enzyme excising the appropriate region of the CSF-1 coding sequence, and the excised sequence ligated into the M13 vector. Second strand synthesis and recovery of the desired mutated DNA used the following oligonucleotide primers:

for $pro_{52}$SCSF, 5'-TACCTTAAACCGGCATTTCTC-3', which creates a new HpaII site at codons 52-53;
for $gln_{52}$SCSF, 5'-TACCTTAAACAGGCCTTTCTC-3', which creates a new StuI site at codons 52-53;
for $asp_{59}$SCSF, 5'-GGTACAAGATATCATGGAG-3', which creates a new EcoRV site at codons 59-60.

After second strand extension using Klenow, the phage were transformed into E coli DG98 and the resulting plaques screened with kinased labeled probe. After plaque purification, the desired mutated inserts were returned to replace the unmutated inserts in pcCSF-1, yielding pCSF-pro52, pCSF-gln52, and pCSF-asp59, respectively.

Plasmids containing three deletion mutants which encode C-terminal deleted SCSF/C∇158 were also prepared: pCSF-Bam, pCSF-BamBcl, and pCSF-BamTGA. For pCSF-Bam, pcCSF-17 was digested with BamHI, and the upstream BamHI/BamHI fragment of the coding region was isolated and religated to the vector fragment. the ligation mixture was transformed into E coli MM294 and plasmids with the correct orientation isolated. The resulting pCSF-Bam encodes 158 amino acids of the CSF-1 protein fused to six residues derived from the vector at the C-terminus: arg-his-asp-lys-ile-his.

For pCSF-BamBcl, which contains the entire CSF-1 encoding sequence, except that the serine at position 159 is mutated to a stop codon, the coding sequence was excised from pcCSF-17 and ligated into M13 for site-specific mutagenesis using the primer: 5'-GAGGGATCCTGATCACCGCAGCTCC-3'. This results in a new BclI site at codons 159-160. The mutated DNA was excised with BstXI/EcoRI and ligated into the BstXI/EcoRI digested pcCSF-17, the ligation mixture was transformed into E coli DG105, a dam host, and the plasmid DNA isolated.

For pCSF-BamTGA, in which the codons downstream of the stop at 159 are deleted, pCSF-BamBcl was digested with XhoI and BclI, and the inserted ligated into XhoI/BamHI digested pcCSF-17.

In addition, pCSF-Gly150, which contains a TGA stop codon instead of histidine at position 151 was prepared from the pcCSF-17 insert by site-specific mutagenesis using the appropriate primer, as described above. The appropriate primer is 5'-AGCCAAGGCTGATCAAGGCAGTCC-3'. Thus, the downstream portion of the coding sequence was excised from pcCSF-17 with BstXI/EcoRI into M13 vectors for mutagenesis, and returned after plaque purification as a BstXI/EcoRI insert.

"Double" muteins were prepared in a similar manner. Thus, for example, for pCSF-asp59-gly150, which encodes $asp_{59}$SCSF/C∇150, pcCSF-asp59 was digested with BstXI/EcoRI to place the C-terminal fragment into M13 for site-specific mutagenesis with the same primer as above for generation of pCSF-gly150. The mutated fragment was then returned to the vector to obtain pCSF-1-asp59-gly150.

Similarly, the procedure for preparation of pCSF-BamBcl was repeated (except that pCSF-asp59 was used instead of pcCSF as a starting plasmid) to obtain pCSF-asp59-BamBclI.

Using the analogous techniques to those described above for preparation of pCSF-gln52 and pCSF-asp59, the corresponding plasmids containing the long forms of the corresponding muteins, pLCSF-gln52 and pLCSF-tyr59, are prepared. The procedure is as described above except that wherever pcCSF-17 is used in that procedure, pcDBhuCSF-4 or its equivalent plasmid is substituted, and the appropriate changes in primer for the $tyr_{59}$ mutein is made.

Site specific mutagenesis is also used to obtain the desired mutations at glycosylation sites of both proteins and for the replacement of cysteine at position 90; it can also be used to obtain various C-terminal deletions of the LCSF-encoding DNA.

F.4. Transient Expression of CSF-1

Expression of pcCSF-17

The expression of plasmid DNA from CSF-17 (pcCSF-17) in COS-7 cells was confirmed and quantitated using the bone marrow proliferation assay, the colony stimulation assay and the radioreceptor assay. It will be recalled that the specificity of the bone marrow proliferation assay for CSF-1 resides only in the ability of CSF-1 antiserum to diminish activity; that for the colony stimulation assay, in the nature of the colonies obtained. Both assays showed CSF-1 production to be of the order of several thousand units per ml.

Bone Marrow Proliferation

For the bone marrow stimulation assay, which measures biological activity of the protein, bone marrow cells from Balb/C mice were treated with serial dilutions of the 72 hour supernatants and proliferation of the cells was measured by uptake of labeled thymidine, essentially as described by Moore, R.N., et al, J Immunol (1983) 131:2374; Prystowsky, M.B., et al, Am J Pathol (1984) 114:149. The medium from induced MIAPaCa cells was used as control. Specificity for CSF-1 was confirmed by the ability of rabbit antisera raised against human urinary CSF-1 to suppress thymidine uptake. The results for COS-7 cell supernatants transfected with pcCSF-17 (CSF-17 supernatant) at a 1:16 dilution are shown in Table 1.

Table 1

| | $^3$H-thymidine incorporation (cpm) | | |
|---|---|---|---|
| | no add'ns | normal serum | antihuman CSF-1 serum |
| medium | 861 | 786 | 2682 |
| MIAPaCa supernate | 12255 | 16498 | 3302 |
| CSF-17 supernate | 16685 | 21996 | 2324 |

(The antihuman CSF-1 serum was prepared as described by Das et al, supra.)

The MIAPcCa supernatant (at the 1:16 dilution used above) contained 125 U/ml CSF activity corresponding to 2000 U/ml in the undiluted supernatant, where 1 unit of colony stimulating activity is defined as the amount of CSF needed to produce one colony from $10^5$ bone marrow cells/ml in the assay of Stanley, E.R., et al, J Lab Clin Med (1972) 79:657.)

These data show that the bone marrow stimulating activity is associated with CSF-1, since thymidine uptake is inhibited by anti-CSF-1 serum. Regression of results in this bone marrow proliferation assay obtained at four dilutions ranging from 1:8 to 1:64 gave an estimated activity for CSF-1 in CSF-17 supernatants of 2358 U/ml, which was diminished to 424 U/ml in the presence of antiserum, but showed an apparent increase to 3693 U/ml in the presence of nonimmune serum. This was comparable to the levels shown in the radioreceptor assay below.

Colony Stimulation

Direct assay of the CSF-17 supernatants for colony stimulation (Stanley, E.R., et al, J Lab Clin Med - (supra) showed 4287 U/ml, which was substantially unaffected by the presence of nonimmune serum but reduced to 0 U/ml in the presence of rabbit antihuman CSF-1. This compares to 2562 U/ml in the MIAPaCa supernatants. Eighty-five percent of the pcCSF-17 transformed COS-7 supernatant induced colonies had mononuclear morphology; MIAPaCa supernatant induced colonies showed a 94% macrophage-6% granulocyte ratio.

Radioreceptor Assay

The radioreceptor assay measures competition between $^{125}$I-labeled CSF-1 and the test compound for specific receptors on J774.2 mouse macrophage cells. MIAPaCa supernatant, assayed for colony stimulating activity as above, was used as a standard (2000 U/ml). The CSF-1 concentration of the pcCSF-17 transformed COS-7 supernatant was found to be 2470 U/ml based on a 1:10 dilution and 3239 U/ml based on a 1:5 dilution.

Thus, comparable values for CSF-1 concentration in the media of COS-7 cells transformed with pcCSF-17 were found in all assays.

Expression of SCSF

In a similar manner to that described above for pcCSF-17, the mutein-encoding plasmids were transfected into COS-A2 cells and transient expression of CSF-1 activity assayed by the bone marrow proliferation assay and by radioimmunoassay using anti-CSF antibodies. The expression product of pCSF-pro52 was inactive, indicating that, as expected, substitution by proline is not conservative. All other muteins showed activity in both assays as shown by the results below:

Table 2

Expression of CSF-1 Constructs in COS Cells

| CSF-1 Plasmid | Radio-immunoassay (units/ml) | Bone Marrow Assay (units/ml) Proliferation (units/ml) | Colony |
|---|---|---|---|
| pcCSF-17 | 3130 | 2798 | 11,100 |
| | 3080 | 3487 | 9750 |
| | 3540 | 3334 | 11,500 |
| pCSF-pro52 | 54.8 | <25 | <100 |
| | 51.9 | <25 | <100 |
| | 45.3 | <25 | <100 |
| pCSF-gln52 | 1890 | 2969 | 6200 |
| | 2250 | 2308 | 5500 |
| | 1910 | 2229 | 4400 |
| pCSF-asp59 | 3210 | 3381 | 9000 |
| | 4680 | 3417 | 6800 |
| | 3470 | 2812 | 10,600 |
| pCSF-Bam | 9600 | 8048 | 22,600 |
| | 8750 | 8441 | 21,900 |
| | 8400 | 10,995 | 21,700 |
| pCSF-BamBcl | 8800 | | 26,000 |
| | 10,700 | | 21,600 |
| | 15,450 | | 24,200 |
| pCSF-BamTGA | 8450 | | 22,600 |
| | 7550 | | 23,200 |
| | 9700 | | 20,000 |
| pCSF-Gly150 | 26,850 | | 55,710 |

Table 3

Mouse Bone Marrow Colony Assay

| CSF-1 Plasmid | | Colony Forming Units/ml |
|---|---|---|
| pCSF-17 | 1 | 25,000 |
| | 2 | 25,000 |
| | 3 | 23,500 |
| pCSF-$asp_{59}gly_{150}$ | 1 | 131,000 |
| | 2 | 125,000 |
| | 3 | 132,000 |
| pCSF-$asp_{59}$BamBcl | 1 | 72,000 |
| | 2 | 78,000 |

Expression of pcDBhuCSF-4 and pcDBhuCSF-25

The Okayama/Berg-type vectors containing the long form of the human CSF-1-encoding DNA (pcDBhuCSF-4 and pcDBhuCSF-25) were transfected into COS-A2 cells in a manner precisely analogous to that described for the transfection of COS-7 cells with pCSF-17 above. The supernatants of the transfected cells were analyzed for CSF-1 using radioimmunoassay with antiserum raised against CSF-1 prepared as described by Das et al (supra), and also in the bone marrow proliferation assay described above. The results, in units of CSF-1 per ml, are shown in Table 4.

Table 4

| Expression of pcDBhuCSF-4 and -25 in COS-A2 cells | | |
|---|---|---|
| Sample | RIA (Units/ml) | BM Assay (Units/ml) |
| pcDBhuCSF-4 | 16,979 | 9,200 |
| pcDBhuCSF-25 | 15,926 | 8,000 |
| medium | 12.5 | <50 |
| Mock infection | 25.0 | <50 |

As indicated, the supernatants of the cells transfected with either vector contained protein with CSF-1 activity.

In a similar manner, the mutein forms of these specific LCSF long form proteins are obtained.

F.5 Eucaryotic Expression of CSF-1 in Stably Transformed Cells

The COS-7 or COS-A2 systems provide recombinant CSF-1 by permitting replication of and expression from the Okayama-Berg-type vector sequences. It is a transient expression system.

The human CSF-1 sequences can also be stably expressed in procaryotic or eucaryotic systems. In general, procaryotic hosts offer ease of production, while eucaryotes permit the use of the native signal sequence to effect secretion and carry out any desired posttranslational processing. This may be important in the case of CSF-1 since the native protein is a dimer. Bacteria produce CSF-1 as a monomer, which could then be subjected to dimerizing conditions after extraction, if needed.

The Okayama-Berg plasmid pcCSF-17, or the analogous vectors containing the DNA encoding muteins, e.g., pCSF-$asp_{59}$-$gly_{150}$ and pCSF-$asp_{59}$-BamBcl, or the analogous vectors pcDBhuCSF-4, pcDBhuCSF-25, pcDBmuCSF-L, and pcDBmuCSF-S, or other vectors encoding muteins of LCSF, containing the cDNA encoding CSF-1 under control of the SV40 promoter can also be used to effect stable expression in monkey CV-1 cells, the parent cell line from which the COS-7 line was derived. The host monkey CV-1 cells are grown to confluence and then cotransformed using 10 μg of the expression vector, and various amounts (1, 2, 5, and 10 μg) of pRSV-NEO2 (Gorman, C., et al, Science (1983) 221:551-553) per 500,000 cells. The transformants are grown in DMEM with 10% FBS medium containing 100 μg/ml of G418 antibiotic, to which the pRSV-NEO2 plasmid confers resistance. The CV-1 cell line shows a G418 transformation frequency of about $10^{-5.12}$ colonies per $10^6$ cells per μg DNA.

The CV-1 cells are contransformed as described above and selected in G418-containing medium. Resistant clones are tested for stability of the G418-resistant phenotype by growth in G418-free medium and then returned to G418-containing medium. The ability of these cultures to survive when returned to antibiotic-containing medium suggests that the pRSV-NEO2 DNA is integrated permanently into the cell genome. Since cells stably transformed with a marker plasmid have about 50% probability of having integrated the DNA of a cotransfecting plasmid, about half of these cells will also contain the expression system for the CSF-1 protein in their chromosomal DNA.

Several clones of the G418-resistant pools of CV-1 cells which are demonstrated to be stably transformed as above are picked and grown in duplicate flasks to near confluence. One flask of each duplicate is infected with SV40 virus at a multiplicity of infection of 5, and the medium is harvested 6 days after infection for assay for CSF-1 using a radioimmunoassay. The immunoassay is based on competition of $^{125}$I-labeled MIAPaCa CSF-1 for "Rabbit 52" polyclonal antiserum raised against purified human urinary CSF-1.

One of the selected CV-1 clones from transfection with pcCSF-17 showed 2335 U/ml production of CSF-1, according to this assay, whereas cells not infected with SV40 showed less than 20 U/ml. Controls using COS-7 cells transformed with 10 μg pcCSF-17 showed 2400 U/ml CSF-1 production without SV40 infection.

The CSF-1-producing CV-1 cell line contains the CSF-1 expression system stably integrated into its genome, and thus can be used for production of CSF-1 upon infection with SV40. Infection is presumed to "rescue" the expression system from the genome, and provide the SV40 T-antigen necessary for replication of the rescued DNA. Without SV40 infection, the integrated gene encoding CSF-1 is not effectively expressed.

Optimization of the expression of the CSF-1 encoding sequence by the CV-1 (CSF-17) cell line showed 6500-8000 U/ml when measured by the radioimmunoassay six days after SV40 infection using a multiplicity of infection of at least 1, and a 10% FBS medium. Studies on expression levels at a multiplicity of 10 showed comparable production, but production was reduced upon removal of the FBS from the medium on the second day after infection.

In the alternative, appropriate control systems and host vectors permitting expression in other eucaryotic hosts may be used to receive the CSF-1 encoding inserts. For example, CHO cells and suitable vectors may be used. In addition, baculovirus vectors containing these sequences are used to infect insect cells for production of protein as described by Miller, D.W., et al, in Genetic Engineering (1986), pp. 277-297 (supra).

F.6 Procaryotic Expression

For procaryotic expression, the cDNA clone, or the genomic sequence with introns excised by, for example, site-specific mutagenesis, is altered to place an ATG start codon immediately upstream of the glutamic acid at the N-terminus, and a HindIII site immediately upstream of the ATG in order to provide a convenient site for insertion into the standard host expression vectors below. This can be done directly using insertion site-specific mutagenesis with a synthetic oligomer containing a new sequence complementary to the desired AAGCTTATG, flanked by nucleotide sequences complementary to the native leader and N-terminal coding sequences.

The DNA fragment containing the entire coding sequence was excised from pcCSF-17, or from pcDBhuCSF-4, by digestion with EcoRI, isolated by agarose gel electrophoresis, and recovered by electroelution. To carry out the mutagenesis, the host bacteriophage M13mp18 DNA was also treated with EcoRI and ligated with the purified fragment under standard conditions and transfected into frozen competent E. coli K12 strain DG98. The cells were plated on media containing 5 x $10^{-4}$ M isopropyl thiogalactoside (IPTG) obtained from Sigma Chem. (St. Louis, MO) and 40 μg/ml X-gal. Noncomplementing white plaques were picked into fresh media. Mini-cultures were screened for recombinant single strand phage DNA of the expected size, and the structure of the desired recombinant phage was confirmed using restriction analysis.

A 34-mer complementary to the N-terminal and leader encoding portions of the CSF-1 sequence, but containing the complement to the desired AAGCTTATG sequence was synthesized and purified. In the alternative, when negative sense strand M13 was used, the positive sense 34-mer was employed. A portion of this 34-mer preparation to be later used as probe was radiolabeled according to a modification of the technique of Maxam and Gilbert (Maxam, A., et al, Methods in Enzymology (1980) 68:521, Academic Press) as set forth above.

To perform the mutagenesis the above prepared recombinant bacteriophage was prepared in E. coli K12 strain DG98 and the single strand phage DNA purified. One pmol of single strand phage DNA and 10 pmol of the above synthetic nucleotide primer (not kinased) was annealed by heating for 1 min at 67°C, and then 30 min at 37°C in 15µl 20 mM Tris-Cl, pH 8, 20 mM $MgCl_2$, 100 mM NaCl, 20 mM 2-mercaptoethanol. The annealed DNA was incubated with DNA polymerase I (Klenow) and 500 µmol dNTPs for 30 min, 0°C and then brought to 37°C. Aliquots (0.05 or 0.25 pmol) were removed after 5 min, 20 min, and 45 min, transformed into E. coli K12 strain DG98 and plated.

After growth, the plates are chilled at 4°C and plaques lifted with Pall membranes obtained from Biodyne or S&S filters (1-2 min in the first filter, more than 10 min for the second filter. The filters are denatured in 2.5 M NaCl, 0.5 M NaOH (5 min). The denaturing medium is neutralized with 3 M sodium acetate to pH 5.5, or with 1 M Tris-Cl, pH 7.5 containing 1 M NaCl, the filters baked at 80°C in vacuo for 1 hr, and then prehybridized at high stringency. The filters are then probed with the kinased synthetic 34-mer prepared above at high stringency, washed, and autoradiographed overnight at -70°C.

The RF form of each desired mutated phage was treated with EcoRI, blunted with Klenow, and then digested with HindIII to excise the gene as a HindIII/blunt fragment.

The plasmid pFC54.t (ATCC 39789), which contains the $P_L$ promoter and the Bacillis thuringiensis positive retroregulatory sequence (as described in EPO Application Publication No. 717,331, published 29 March 1985), was used as a host vector. pFC54.t was digested with HindIII/BamHI(blunt), and the desired coding sequences were ligated into the vector using the HindIII/EcoRI(blunt) excised fragment from the mutated M13 derived from pcCSF-17 or pcDBhuCSF-4. After transformation into E. coli MC1000 lambda lysogen, and induction, CSF-1 production, putatively of SCSF and LCSF, respectively, was obtained and verified as described above.

Thus, for example, the HindIII/EcoRI blunt fragment from the M13 substrate mutated from pcCSF-17 was ligated into pFC54.t as described above to obtain $pP_L$CSF-17. $pP_L$CSF-17 was then digested with BamHI and religated to obtain $pP_L$CSF-17/C∇158, which contains the coding sequence for mature SCSF preceded by an ATG start codon, and truncated so as to encode a protein having the first 158 amino acids of the SCSF sequence, followed by an in-reading-frame proline and stop codon from the retroregulatory sequence of pFC54.t. $pP_L$CSF-17/C∇158 was further modified by site-specific mutagenesis. The HindIII/BamHI coding sequence was excised and ligated into M13. An appropriate primer was used to place a stop codon immediately after the codon encoding amino acid 158, and to follow it by TGA. This creates a BamHI/BclI site for further manipulation. The mutated fragment was then excised from M13 and religated into HindIII/BamHI-digested $pP_L$CSF-17/C∇158 to obtain $pP_L$CSF-17/C∇158TGA.

Other constructs having codons for substitute amino acids include modifications replacing lys at position 52 by gln or pro and/or tyr at 59 by asp in SCSF. These constructs were made analogously by site-directed mutagenesis of M13 inserts containing the HindIII/EcoRI (blunt) fragment from the original $pP_L$CSF-17. HindIII/EcoRI fragments from these mutated phages are then used in place of the original HindIII/EcoRI insert in construction of corresponding vectors. In general, desired mutations in selected locations can be made according to variations of this standard technique.

It is possible to improve the level of CSF-1 production from any of the foregoing constructs by altering the third nucleotide in each of the first six codons of the N-terminus. pFC54.t containing the CSF-1 encoding fragment of any mutein is digested with HindIII/BstXI, and the excised fragment (which contains the ATG and a short portion of the subsequent coding sequence) is replaced by a synthetic HindIII/BstXI segment wherein the first six codons have the sequence: GAAGAAGTTTCT GAATAT. The resulting analogous expression vector represents no change in the amino acid sequence encoded; however, the levels of expression were improved when this modified vector was used. The corresponding vectors are prefixed by O/E (over-expression). Thus, for example, O/E $pP_L$CSF-17, O/E $pP_L$CSF-17/C∇158, and O/E $pP_L$CSF-17/C∇158TGA are formed.

The corresponding vector, O/E $pP_L$CSF-17$asp_{59}$/C∇158TGA was synthesized as described above except that the M13 containing the original EcoRI insert was doubly mutated to place a HindIII site and ATG start codon immediately before amino acid 1 of the mature protein, and to replace the tyrosine codon at residue 59 by a codon representing aspartic acid. The corresponding vectors O/E $pP_L$CSF-17$asp_{59}$/C∇158, and O/E $pP_L$CSF-17$asp_{59}$/C∇158TGA are thus correspondingly formed. In all of the foregoing cases, each of the intermediate vectors can be digested with HindIII and BstXI and the synthetic DNA fragment containing preferred codons substituted for the native sequence encoding the first six amino acids.

All of the foregoing vectors are modified to encode proteins with the N-terminal deletion characteristic of the invention by substitution of the appropriate synthetic fragment for the excised HindIII/BstXI DNA. In this manner, the corresponding vectors encoding CSF/N∇2 and CSF/N∇3, lacking glu-glu and glu-glu-val were constructed.

Analogous vectors were also constructed containing a termination codon immediately after the glycine residue at position 150 of the SCSF protein. These vectors were obtained by mutagenesis of the HindIII/EcoRI fragment from $pP_LCSF$-17 or $pP_LCSF$-17$asp_{59}$ or their corresponding muteins into HindIII/SmaI-digested M13. The resulting vectors, for example, $pP_LCSF$-17/C∇150 and $pP_LCSF$-17$asp_{59}$/C∇150 encode, therefore, the first 150 amino acid residues of the mature SCSF protein.

The foregoing vectors when transformed into E. coli λ lysogen host, such as DG116 and induced at high temperature (approximately 42°C) produce CSF-1 and muteins thereof in monomeric or aggregated deglycosylated form. The production of this protein can be detected by the use of standard radioimmunoassay or other CSF-1 specific assays. The activity of the protein can be measurably improved by the use of refolding procedures.

Constructs have also been made using the phosphatase A promoter and secretory leader sequence in place of the $P_L$ promoter. The control sequences, including the 3' retroregulatory sequences corresponding to those in pFC54.t are provided by inserting the desired coding sequence into NarI/BamHI digested pSYC1089, a host vector which contains these controls.

In order to utilize this vector, the relevant CSF-encoding sequences are religated into M13 to change the HindIII site immediately preceding the ATG to a ClaI site. The mutated sequence is then excised as a ClaI/BclI fragment and ligated into the digested pSYC1089 vector as described. The products of this construct, corresponding to the short forms produced under the control of the $P_L$ promoter in the $P_L$ vector series are secreted into the periplasmic space when expressed in E. coli and are relatively soluble as compared to products of genes expressed under $P_L$ control. Vectors which can be constructed using the phoA system here described include pPhoA-LCSF/C∇158 and pPhoA/N∇3C∇150. These vectors are transformed for expression into a non-λ lysogen host, such as E. coli MM294.

Vectors were also constructed using DNA derived from the long form clone. O/E $pP_LCSF$-17$asp_{59}$/C∇150, described above, was used as the host vector for constructs of the long form and of various C-terminal truncations in the coding sequence for the long form. Because the sequences upstream from the BstXI site are common to both the short and long forms, vectors containing the short form and its muteins can be converted to long form vectors by interchanging the BstXI to BamHI fragment of this vector, which excises the downstream portions of the sequence through and including the TGA stop codon with the BstXI to BamHI fragment of M13 containing the long-form coding sequence from clone 4, or its C-terminal truncations.

Accordingly, the M13 clone containing the clone 4 insert was mutagenized with appropriate oligomers to insert stop codons after the residues at positions after 150, 190 191, 223, 236, 238, 249, 258, and 411. For example, the reverse strand M13 containing the clone 4 insert was primed with the oligonucleotide GGCTTGACCTGATCAGACTCTGAGG in order to place a stop codon after the threonine residue at position 191 and provide a unique BclI site immediately thereafter. The mutagenized RF form of the phage was digested with BstXI and BamHI and ligated into BstXI/BamHI-digested O/E $pP_LCSF$-17$asp_{59}$/C∇150 to give O/E $pP_LLCSFasp_{59}$/C∇150.

The mutated long form can be used to replace the corresponding short form fragments from vectors encoding CSF-1 proteins with N-terminal deletions, as well as from the corresponding constructions in the phoA-controlled vectors. For example, pPhoA-LCSF/N∇3C∇221and pPhoA-LCSF/C∇221 were constructed and transformed into E. coli MM294. Of course, more than one mutation of the long form can be effected; also, amino acid substitutions, such as $gly_{52}$ or $tyr_{59}$, can be made.

Additional constructs have been made for muteins encoding SCSF, LCSF and their muteins wherein the glycosylation sites at positions 122-124 and 140-142, common to both proteins have been replaced with amino acids not subject to glycosylation. The CSF proteins produced from bacterial expression of these constructions are active in biological assays; this substantiates the proposition that glycosylation is not necessary for activity. However, replacement of any of the cysteine residues between positions 1-150 by serine or alanine results in inactive proteins, except that muteins having a substitution at position 90 have immunoreactivity. Evidently all seven of these cysteines are required, or at least desirable, for proper refolding.

Construction of N∇2 and N∇3 muteins of the invention from any of the above vectors is extremely straightforward, and simply involves deleting the HindIII/BstXI fragment and replacing it with a synthetic fragment which does not include the codons for the first two or three amino acids, as the case may be. Thus, each of the foregoing vectors is modified to obtain a vector encoding the muteins of the invention by this simple substitution procedure.

Included in the recombinant organisms prepared are E. coli DG116 λ lysogen transformed with the following plasmids, which are constructs for the expression of the indicated short form-derived CSF-1 mutein forms:

| Construct | Encoded Protein |
|---|---|
| $pP_L$CSF-17 | SCSF |
| $pP_L$CSF-17$gln_{52}$ | $gln_{52}$SCSF |
| $pP_L$CSF-17$pro_{52}$ | $pro_{52}$SCSF |
| $pP_L$CSF-17/C∇158 | SCSF/C∇158-pro |
| $pP_L$CSF-17$gln_{52}$/C∇158 | $gln_{52}$SCSF/C∇158-pro |
| $pP_L$CSF-17$pro_{52}$/C∇158 | $pro_{52}$SCSF/C∇158-pro |
| $pP_L$CSF-17$asp_{59}$ | $asp_{59}$SCSF |
| $pP_L$CSF-17/C∇158TGA | SCSF/C∇158 |
| O/E $pP_L$CSF-17/C∇158TGA | SCSF/C∇158 |
| $pP_L$CSF-17$pro_{52}$/C∇158TGA | $pro_{52}$SCSF/C∇158 |
| $pP_L$CSF-17$asp_{59}$/C∇158TGA | $asp_{59}$SCSF/C∇158 |
| O/E $pP_L$CSF-17$asp_{59}$/C∇158TGA | $asp_{59}$SCSF/C∇158 |
| $pP_L$CSF-17/C∇150 | SCSF/C∇150 |
| O/E $pP_L$CSF-17/C∇150 | SCSF/C∇150 |
| $pP_L$CSF-17$asp_{59}$/C∇150 | $asp_{59}$SCSF/C∇150 |
| O/E $pP_L$CSF-17$asp_{59}$/C∇150 | $asp_{59}$SCSF/C∇150 |
| O/E $pP_L$CSF-17$asp_{59}$/N∇2C∇150 | $asp_{59}$SCSF/N∇2C∇150 |
| O/E $pP_L$CSF-17$asp_{59}$/N∇3C∇150 | $asp_{59}$SCSF/N∇3C∇150 |

The following analogous recombinant organisms encoding variations of the long form are constructed:

| | |
|---|---|
| O/E $pP_L$LCSF/C∇150 | LCSF/C∇1/C∇150 |
| O/E $pP_L$LCSF$gln_{52}$/C∇150 | $gln_{52}$LCSF/C∇150 |
| O/E $pP_L$LCSF$tyr_{59}$/C∇190 | $tyr_{59}$LCSF/C∇190 |
| O/E $pP_L$LCSF/C∇191 | LCSF/C∇191 |
| O/E $pP_L$LCSF/N∇2C∇191 | LCSF/N∇2C∇190 |
| O/E $pP_L$LCSF/C∇221 | LCSF/C∇221 |
| O/E $pP_L$LCSF/N∇3C∇221 | LCSF/N∇3C∇221 |
| O/E $pP_L$LCSF/C∇223 | LCSF/C∇223 |
| O/E $pP_L$LCSF/C∇236 | LCSF/C∇236 |
| O/E $pP_L$LCSF/C∇238 | LCSF/C∇238 |
| O/E $pP_L$LCSF/C∇249 | LCSF/C∇249 |
| O/E $pP_L$LCSF/C∇250 | LCSF/C∇250 |
| O/E $pP_L$LCSF/C∇411 | LCSF/C∇411 |
| phoA-LCSF/C∇221 | LCSF/C∇221 |
| phoA-LCSF/N∇3C∇221 | LCSF/N∇3C∇221 |
| O/E phoA-LCSF/C∇221 | LCSF/C∇221 |
| O/E phoA-LCSF/N∇3C∇221 | LCSF/N∇3C∇221 |

For expression of the constructions, suitably transformed E. coli (λ lysogen, e.g., DG116 for the $P_L$ constructs, non-lysogen, e.g., MM294 for PhoA) are grown to the desired cell density and then production of the encoded protein induced. If expressed under control of the $P_L$ promoter, an increase in temperature to 37°C or 42°C induces expression. The addition of about 0.5-2% casamino acids to the medium is also helpful in increasing expression. The constructs described above result in the formation of CSF-1 as an insoluble intracellular protein which can be recovered from the lysed cells, purified and refolded. Similar procedures can be used to purify and refold the CSF-1 secreted to the periplasmic space of E. coli transformed with the phoA-controlled genes.

In general, the refolding begins with the solubilized monomer in a chaotropic environment, which is maintained under reducing conditions. Such maintenance may involve the use of a suitable reducing agent such as β-mercaptoethanol or dithiothreitol (DTT) but the CSF-1 may already by reduced, the exclusion of oxidizing agents may be sufficient. The solubilized protein is typically maintained in, for example, 8 M urea or 7 M guanidinium hydrochloride, at a pH of about 7-8.5, in the presence of about 25-100 mM thiol compound. Starting with this solubilized form, the monomer may either be refolded directly or purified from remaining proteins by a suitable purification procedure such as chromatography on an adsorbant gel or gel-permeation chromatography prior to refolding. Gel-permeation chromatography is preferred, as it permits an easy size separation of the desired monomer length, which is generally known in advance, from impurities of differing molecular weights. It is required that the purification be conducted under reducing conditions in order to prevent the formation of disulfide-linked aggregates. Thus, regardless of the chromatographic procedure used, a suitable reducing agent is preferably included in the solutions used to load the chromatographic columns or batches and in the eluting solutions. In some instances, low pH may be substituted for reducing agent, as low pH will prevent disulfide bond formation in some chromatographic systems, even in the absence of reducing agent.

The partially purified monomer is then subjected to refolding conditions for the formation of the dimer. The protein concentration during this step is of condierably importance. Generally, yields are increased if the protein concentration is less than 2 mg/ml of the CSF-1 protein; a concentration range of 0.03-0.3 mg/ml is preferred. The refolding conditions may include gradual removal of the chaotropic environment over an appropriate time period, usually several hours. One easy method is dilution of the sample to the desired concentration of the chaotropic agent, but this may not in certain instances be preferred, since the protein also becomes diluted. More preferred are method which provide a constant protein concentration, such as dialysis or hollow fiber diafiltration. At the end of the process, when the chaotropic environment is depleted, a nondenaturing level is reached. For example, if guanidine hydrochloride is used as chaotropic agent, a final concentration of less than about 2 M, and preferably 0.5-1 M is attained.

The refolding during removal of chaotropic environment is conducted in a manner so as to permit oxoidation of the sulfhydryl groups to disulfides in order to establish the resultant biologically active dimeric configuration which, in the case of CSF-1 is stabilized by the formation of disulfides, one or more of which may link the two chains. Intrachain disulfides are also formed. Suitable redox conditions which encourage this formation of dimer include the SH/disulfide reagent combinations, such as oxidized and reduced glutathione. The ratio of reduced to oxidized glutathione or other sulfhydryl/disulfide combination is typically from about 2 mM/0.1 mM to 0.5 mM/1.0 mM. Alternative methods for providing this oxidation are also acceptable. Simple removal of the reducing agent without precautions to exclude air and metal ions may suffice to effect formation of some correct disulfide linkages, but this is less preferred. In any event, the pH of the solution during the refolding process should be maintained at about pH 7.5-9.0. It is clear that in the process of refolding, the reducing conditions under which the initial purification was conducted are no longer employed.

During the refolding process, aggregates of the monomer, which are insoluble, may form. This is minimized through temperature control, wherein low temperatures of about 0-4 °C are preferable to higher temperatures of 25-37 °C. After refolding is completed, the dimer is purified from other proteins using standard procedures.

When the constructs of the invention encoding the N-terminal depleted muteins are expressed in E.coli as mentioned above, the methionine at the N-terminus is processed more efficiently than is the case for the corresponding mature sequences. Specifically, expression of $pP_L$CSF-17/C∇150 results in protein wherein most, if not all of the sequenceable molecules contain N-terminal methionine; corresponding expression of $pP_L$CSF-17/N∇2C∇150 results in protein wherein only 78% of the molecules retain the N-terminal methionine. Expression of $pP_L$CSF-17/N∇3C∇150 gives protein wherein only about 5% of the product contains methionine at the N-terminus.

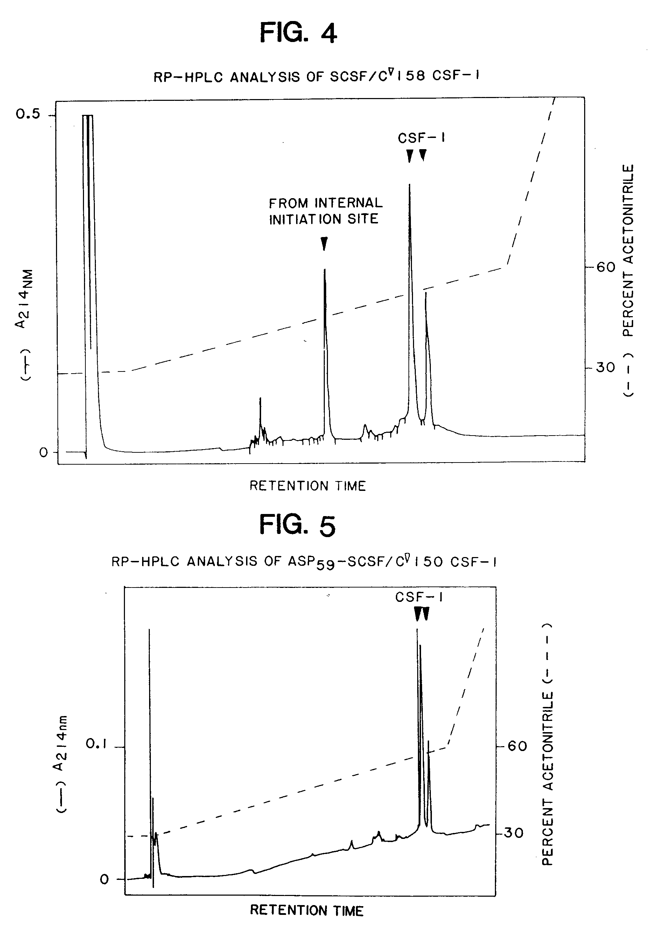

Figures 4-6 show the effect of N-terminal deletions on resulting protein heterogeneity. Figures and 5 show the RP-HPLC analyses of purified CSF-1 expressed in E. coli using $pP_L$CSF-17/C∇158 ($tyr_{59}$) and $pP_L$CSF-17/C∇150 ($asp_{59}$), respectively. As shown in both figures, there are two major peaks at approximately 18 kd in the reducing conditions under which these analyses were run. The leading peak, labeled 18 K-A in Figure 4 (and the corresponding peak in Figure 5) comprises approximately 70% of the total of the 18 kd protein, and has essentially the same amino acid composition and SDS-PAGE subunit molecular weight as the trailing peak (labeled 18 K-B). In addition, the 158 codon construct results in a major impurity in the form of a 15 kd protein, which is evidently the product of an internal restart. This peak seems to be missing from the product of the 150 codon ($asp_{59}$) construct, shown in Figure 5.

The results in Figure 5 should properly be compared with those of Figure 6, which represent RP-HPLC analysis from the expression of

$pP_L$CSF-17/N▽3C▽150,

The significant heterogeneity seen on RP-HPLC has disappeared, and a single peak corresponding to the leading 70% peak of Figure 5 is obtained.

G. Formulation of CSF-1

The recombinantly produced human CSF-1 short or long form may be formulated for administration using standard pharmaceutical procedures. Ordinarily CSF-1 will be prepared in injectable form, and may be used either as the sole active ingredient, or in combination with other proteins or other compounds having complementary or similar activity. Such other compounds may include alternative antitumor agents such as adriamycin, or lymphokines, such as IL-1, -2, and -3, alpha-, beta-, and γ-interferons and tumor necrosis factor. The effect of the CSF-1 active ingredient may be augmented or improved by the presence of such additional components. As described above, the CSF-1 may interact in beneficial ways with appropriate blood cells, and the compositions of the invention therefore include incubation mixtures of such cells with CSF-1, optionally in the presence of additional lymphokines. Either the supernatant fractions of such incubation mixtures, or the entire mixture containing the cells as well may be used.

Deposits

On 2 April 1985, Applicants have deposited with the American Type Culture Collection, Rockville, MD, USA (ATCC) the phage phCSF-1 in E. coli dG98, accession no. 40177. On 21 May 1985, phCSF-1a, designated CMCC 2312 in the Cetus collection, designated phCSF-1a/λ Charon 4A for deposit, was deposited with ATCC and has accession no. 40185. On 14 June 1985, pcCSF-17 in E coli MM294, designated CMCC 2347, was deposited with ATCC and has accession no. 53149.

In addition, pcDBCSF4, designated herein pcdBhuCSF-4 (CMCC 2894), was deposited with ATCC on 24 October 1986 and has ATCC accession no. 67250. On 7 April 1987, $pP_L$CSF-17$asp_{59}$/C▽150 in DG116 (CMCC 2946) was deposited at ATCC and has accession number 67,383.

Additional deposits were made on 14 April 1987 as follows:

| Strain | CMCC No. | ATCC No. |
|---|---|---|
| pPhoA-LCSF/C▽221 in MM294 | 3084 | 67,391 |
| O/E $pP_L$LCSF/N▽3C▽221 in DG116 | 3095 | 67,390 |
| O/E $pP_L$CSF-17$asp_{59}$/C▽150 in DG116 | 3044 | 67,389 |

These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years form date of deposit. The deposits will be made available by ATCC under the terms of the Budapest treaty, and subject to an agreement between Applicants and ATCC which assures permanent and unrestricted availability upon issuance of the pertinant US patent. The Assignee herein agrees that if the culture on deposit die or be lost or destroyed when cultivated under suitable conditions, it will be promptly replaced upon notification with a viable speciment of the same culture. Availability of the deposits is not to be construed as a license to practice the invention in contravention of the rights granted under the authority of any government in accordance with its patent laws.

These deposits were made for the convenience of the relevent public and do not constitute an admission that a written description would not be sufficient to permit practice of the invention or an intention to limit the invention to these specific constructs. Set forth hereinabove is a complete written description enabling a practitioner of ordinary skill to duplicate the constructs deposited and to construct alternative forms of DNA, or organisms containing it, which permit practice of the invention as claimed.

The scope of the invention is not to be construed as limited by the illustrative embodiments set forth herein, but is to be determined in accordance with the appended claims.

## Claims

1. A DNA sequence which encodes for a human CSF-1 N∇3 or N∇2 mutein having at least the amino acid sequence of:
   (i) N∇3 or N∇2/SCSF/C∇150; or of
   (ii) N∇3 or N∇2/LCSF/C∇150; or of
   (iii) a modification of either (i) or (ii) in which 1-5 amino acids are substituted or deleted, and wherein dimers of said mutein stimulate the formation of primarily macrophage colonies in the in vitro CSF-1 assay; and wherein the term "SCSF" refers to short form CSF, the term "LCSF" refers to long form CSF and the term "∇" indicates deletion of one or more amino acids at the "N" terminal or truncation of the molecule by deletion of subsequent amino acids at the "C" terminal.

2. A DNA sequence of claim 1, wherein said DNA sequence encodes a CSF-1 polypeptide further comprising the amino acid sequence shown as amino acids 150-447 of Figure 2.

3. A DNA sequence of claim 1, wherein said DNA sequence encodes a CSF-1 polypeptide which is an N∇3 mutein of any one of the following:-
   SCSF, $gln_{52}$SCSF, $pro_{52}$SCSF, SCSF/C∇158-pro, $gln_{52}$SCSF/C∇158-pro, $pro_{52}$SCSF/C∇158-pro, SCSF/C∇158, $pro_{52}$SCSF/C∇158, SCSF/C∇150, LCSF/C∇150, $gln_{52}$LCSF/C∇150, $tyr_{59}$LCSF/C∇190, LCSF/C∇191, LCSF/C∇221, LCSF/C∇223, LCSF/C∇236, LCSF/C∇238, LCSF/C∇249, LCSF/C∇250, and LCSF/C∇411.

4. A DNA sequence of claim 1, wherein said DNA sequence encodes a CSF-1 polypeptide selected from:
   (a) N∇3 muteins of LCSF, SCSF, LCSF/C∇150, $tyr_{59}$LCSF/C∇150, SCSF/C∇150 and $gln_{52}$ muteins thereof;
   (b) N∇3 muteins of each of the possible C-terminal deletion polypeptides from LCSF/C∇149 to LCSF/C∇521, and $tyr_{59}$, $gln_{52}$ and $tyr_{59}gln_{52}$ muteins thereof;
   (c) N∇3 muteins of LCSF/C∇190, LCSF/C∇191, LCSF/C∇221, LCSF/C∇223, LCSF/C∇236, LCSF/C∇238, LCSF/C∇249, LCSF/C∇258 and LCSF/C∇411 and $tyr_{59}$, $gln_{52}$, $tyr_{59}gln_{52}$, $ser_{157}$, $ser_{159}$, $ser_{157}ser_{159}$ muteins thereof; and
   (d) N∇3 muteins of SCSF/C∇158 and the $gln_{52}$ muteins thereof.

5. A DNA sequence of claim 1, wherein said DNA sequence encodes a polypeptide comprising LCSF/N∇3C∇221 or LCSF$ser_{157}ser_{159}$N∇3C∇221 or SCSF/N∇3C∇158.

6. A vector which comprises a DNA sequence of any one of claims 1 to 5 and a replicon operative in a unicellular organism.

7. A vector of claim 6 comprising O/Ep$P_L$LCSF/N∇3C∇221 obtainable from ATCC 67390.

8. An expression system which comprises a DNA sequence of any one of claims 1 to 5 or a vector of claim 6 or claim 7 operably linked to suitable control sequences.

9. Recombinant host cells transformed with an expression system of claim 8.

10. A method of producing a recombinant human CSF-1 polypeptide, which comprises culturing cells of claim 9 under conditions effective for the production of said CSF-1.

## Patentansprüche

1. DNA-Sequenz, die ein menschliches CSF-1- N∇3- oder -N∇2-Mutein codiert, das mindestens die Aminosäuresequenz:
   (i) N∇3 oder N∇2/SCSF/C∇150; oder
   (ii) N∇3 oder N∇2/LCSF/C∇150; oder
   (iii) eine Modifikation von (i) oder (ii) aufweist, wobei 1 bis 5 Aminosäuren substituiert oder deletiert sind und wobei Dimere des Muteins die Bildung von primären Makrophagenkolonien im in vitro-CSF-1-Test stimulieren; und wobei der Begriff "SCSF" sich auf die CSF-Kurzform bezieht, der Begriff "LCSF" sich auf die CSF-Langform bezieht und der Begriff "∇" die Deletion einer oder mehrerer Aminosäuren am N-terminalen Ende oder die Verkürzung des Moleküls durch Deletion von aufeinanderfolgenden Aminosäuren am C-terminalen Ende bedeutet.

**2.** DNA-Sequenz nach Anspruch 1, wobei die DNA-Sequenz ein CSF-1-Polypeptid codiert, das außerdem die Aminosäuresequenz umfaßt, die als Aminosäuren 150-447 in Figur 2 gezeigt ist.

**3.** DNA-Sequenz nach Anspruch 1, wobei die DNA-Sequenz ein CSF-1-Polypeptid codiert, welches eines der folgenden N∇3-Muteine ist:
SCSF, $gln_{52}$SCSF, $pro_{52}$SCSF, SCSF/C∇158-pro,$gln_{52}$SCSF/C∇158-pro, $pro_{52}$SCSF/C∇158-pro, SCSF/C∇158, $pro_{52}$SCSF/C∇158, SCSF/C∇150, LCSF/C∇150, $gln_{52}$LCSF/C∇150, $tyr_{59}$LCSF/C∇190, LCSF/C∇191, LCSF/C∇221, LCSF/C∇223, LCSF/C∇236, LCSF/C∇238, LCSF/C∇249, LCSF/C∇250 und LCSF/C∇411.

**4.** DNA-Sequenz nach Anspruch 1, wobei die DNA-Sequenz ein CSF-1-Polypeptid codiert, das ausgewählt ist aus:
(a) N∇3-Muteinen von LCSF, SCSF, LCSF/C∇150, $tyr_{59}$LCSF/C∇150, SCSF/C∇150 und $gln_{52}$-Muteinen davon;
(b) N∇3-Muteinen von jedem der möglichen C-terminalen Deletionspolypeptiden von LCSF/C∇149 bis LCSF/C∇521 und $tyr_{59}$-, $gln_{52}$- und $tyr_{59}gln_{52}$-Muteinen davon;
(c) N∇3-Muteinen von LCSF/C∇190, LCSF/C∇191, LCSF/C∇221, LCSF/c∇223, LCSF/C∇236, LCSF/C∇238, LCSF/C∇249, LCSF/C∇258 und LCSF/C∇411 und $tyr_{59}$-, $gln_{52}$-, $tyr_{59}gln_{52}$-, $ser_{157}$-, $ser_{159}$-, $ser_{157}ser_{159}$-Muteinen davon; und
(d) N∇3-Muteinen von SCSF/C∇158 und den $gln_{52}$-Muteinen davon.

**5.** DNA-Sequenz nach Anspruch 1, wobei die DNA-Sequenz ein Polypeptid codiert, welches LCSF/N∇3C∇221 oder LCSF$ser_{157}ser_{159}$N∇3c∇221 oder SCSF/N∇3C∇158 umfaßt.

**6.** Vektor, umfassend eine DNA-Sequenz nach einem der Ansprüche 1 bis 5 und ein Replicon, das in einem einzelligen Organismus wirksam ist.

**7.** Vektor nach Anspruch 6, umfassend O/Ep$P_L$LCSF/N∇3C∇221, erhältlich aus ATCC 67390.

**8.** Expressionssystem, umfassend eine DNA-Sequenz nach einem der Ansprüche 1 bis 5 oder einen Vektor nach Anspruch 6 oder Anspruch 7, funktionell verknüpft mit geeigneten Kontrollsequenzen.

**9.** Rekombinante Wirtszellen, die mit einem Expressionssystem nach Anspruch 8 transformiert sind.

**10.** Verfahren zur Herstellung eines rekombinanten menschlichen CSF-1-Polypeptids, umfassend die Züchtung von Zellen nach Anspruch 9, unter Bedingungen, die die Produktion des CSF-1 bewirken.

**Revendications**

**1.** Séquence d'ADN qui code pour une mutéine N∇3 ou N∇2 du CSF-1 possédant au moins la séquence d'acides aminés du :
(i) $N\nabla_3$ ou $N\nabla_2$/SCSF/C△150, ou du
(ii) $N\nabla_3$ ou $N\nabla_2$/LCSF/C△150, ou
(iii) une modification de (i) ou de (ii) dans laquelle 1-5 acides aminés sont substitués ou supprimés et dans laquelle des dimères de ladite mutéine stimulent la formation de colonies essentiellement composées de macrophages lors du dosage in vitro du CSF-1; et dans laquelle le terme "SCSF" s'applique à une forme courte de CSF, le terme "LCSF" s'applique à une forme longue de CSF et le symbole "∇" indique une délétion d'un ou plusieurs acides aminés à l'extrémité "N" ou la formation d'une extrémité tronquée de la molécule par délétion des acides aminés suivants la position indiquée à l'extrémité "C".

**2.** Séquence d'ADN selon la revendication 1, dans laquelle ladite séquence d'ADN code pour un CSF-1 de nature polypeptidique et comprend en outre la séquence d'acides aminés représentée par les acides aminés 150 à 447 à la figure 2.

**3.** Séquence d'ADN selon la revendication 1, dans laquelle ladite séquence d'ADN code pour un CSF-1 de nature polypeptidique qui est une mutéine N▽3 ou l'une des mutéines suivantes:
SCSF, SCSFgln$_{52}$, SCSFpro$_{52}$, SCSF/C▽158-pro, SCSFgln$_{52}$/C▽158-pro, SCSFpro$_{52}$/C▽158-pro, SCSF/C▽158, SCSFpro$_{52}$/C▽158, SCSF/C▽150, LCSF/C▽150, LCSFgln$_{52}$/C▽150, LCSFtyr$_{59}$/C▽190, LCSF/C▽191, LCSF/C▽ 221, LCSF/C▽223, LCSF/C▽236, LCSF/C▽238, LCSF/C▽249, LCSF/C▽250 et LCSF/C▽411.

**4.** Séquence d'ADN selon la revendication 1, dans laquelle ladite séquence d'ADN code pour un CSF-1 de nature polypeptidique choisi parmi:
(a) les mutéines N▽3 du LCSF, du SCSF, du LCSF/C▽150, du LCSFtyr$_{59}$/C▽ 150, du SCSF/C▽150 et de leurs mutéines en gln$_{52}$;
(b) les mutéines N▽3 de tous les polypeptides possibles portant une délétion à l'extrémité C issus du LCSF/C▽149 au LCSF/C▽521 et de leurs mutéines en tyr$_{59}$, en gln$_{52}$ et en tyr$_{59}$gln$_{52}$;
(c) les mutéines N▽3 du LCSF/C▽190, LCSF/C▽191, LCSF/C▽221, LCSF/C▽ 223, LCSF/C▽236, LCSF/C▽238, LCSF/C▽249, LCSF/C▽258 et LCSF/C▽411 et de leurs mutéines en tyr$_{59}$, en gln$_{52}$, en tyr$_{59}$gln$_{52}$, ser$_{157}$, en ser$_{159}$, et en ser$_{157}$ser$_{159}$; et
(d) les mutéines N▽3 du SCSF/C▽158 et de ses mutéines en gln$_{52}$.

**5.** Séquence d'ADN selon la revendication 1, dans laquelle ladite séquence d'ADN code pour un polypeptide comprenant le LCSF/N▽3C▽221 ou le LCSFser$_{157}$ser$_{159}$/N▽3C▽221 ou le SCSF/N▽3C▽158.

**6.** Vecteur comprenant une séquence d'ADN selon l'une des revendications de 1 à 5 et un réplicon opérationnel dans un organisme unicellulaire.

**7.** Vecteur selon la revendication 6 qui comprend l'O/ExpP$_L$LCSF/N▽ 3C▽221 disponible auprès de l'ATCC sous le numéro 67390.

**8.** Système d'expression qui comprend une séquence d'ADN selon l'une des revendications 1 à 5 ou un vecteur selon la revendication 6 ou 7 liés de manière opérationnelle avec des séquences de régulation appropriées.

**9.** Cellules hôtes recombinantes transformées avec un système d'expression selon la revendication 8.

**10.** Procédé de production d'un CSF-1 humain de nature polypeptidique qui comprend la culture de cellules selon la revendication 9 dans des conditions efficaces pour la production dudit CSF-1.

# FIG. 1-1

```
                    20                  40                  60
AGTGAGGCTC GGCCCGGGGA AAGTGAAAGT TTGCCTGGGT CCTCTCGGCG CCAGAGCCGC

                    80                 100                 120
TCTCCGCATC CCAGGACAGC GGTGCGGCCC TCGGCCGGGG CGCCCACTCC GCAGCAGCCA
                                             ▲
                   140                 160
GCGAGCGAGC GAGCGAGCGA GGGCGGCCGA CGCGCCCGGC CGGGACCCAG CTGCCCGT

180                             200                             220
ATG ACC GCG CCG GGC GCC GCC GGG CGC TGC CCT CCC ACG ACA TGG CTG GGC
Met Thr Ala Pro Gly Ala Ala Gly Arg Cys Pro Pro Thr Thr Trp Leu Gly
-32

            240                             260
TCC CTG CTG TTG TTG GTC TGT CTC CTG GCG AGC AGG AGT ATC ACC GAG
Ser Leu Leu Leu Leu Val Cys Leu Leu Ala Ser Arg Ser Ile Thr Glu
                                                            1

280                         300                             320
GAG GTG TCG GAG TAC TGT AGC CAC ATG ATT GGG AGT GGA CAC CTG CAG TCT
Glu Val Ser Glu Tyr Cys Ser His Met Ile Gly Ser Gly His Leu Gln Ser

            340                             360
CTG CAG CGG CTG ATT GAC AGT CAG ATG GAG ACC TCG TGC CAA ATT ACA
Leu Gln Arg Leu Ile Asp Ser Gln Met Glu Thr Ser Cys Gln Ile Thr
    20  INTRON ▲ SEQUENCE

380                         400                             420
TTT GAG TTT GTA GAC CAG GAA CAG TTG AAA GAT CCA GTG TGC TAC CTT AAG
Phe Glu Phe Val Asp Gln Glu Gln Leu Lys Asp Pro Val Cys Tyr Leu Lys
                        40

            440                         460
AAG GCA TTT CTC CTG GTA CAA TAC ATA ATG GAG GAC ACC ATG CGC TTC
Lys Ala Phe Leu Leu Val Gln Tyr Ile Met Glu Asp Thr Met Arg Phe
                            *   60

480                         500                             520
AGA GAT AAC ACC CCC AAT GCC ATC GCC ATT GTG CAG CTG CAG GAA CTC TCT
Arg Asp Asn Thr Pro Asn Ala Ile Ala Ile Val Gln Leu Gln Glu Leu Ser
                                            80

            540                         560
TTG AGG CTG AAG AGC TGC TTC ACC AAG GAT TAT GAA GAG CAT GAC AAG
Leu Arg Leu Lys Ser Cys Phe Thr Lys Asp Tyr Glu Glu His Asp Lys
                                                            100

580                         600                             620
GCC TGC GTC CGA ACT TTC TAT GAG ACA CCT CTC CAG TTG CTG GAG AAG GTC
Ala Cys Val Arg Thr Phe Tyr Glu Thr Pro Leu Gln Leu Leu Glu Lys Val

            640                         660
AAG AAT GTC TTT AAT GAA ACA AAG AAT CTC CTT GAC AAG GAC TGG AAT
Lys Asn Val Phe Asn Glu Thr Lys Asn Leu Leu Asp Lys Asp Trp Asn
    120

680                         700                             720
ATT TTC AGC AAG AAC TGC AAC AAC AGC TTT GCT GAA TGC TCC AGC CAA GGC
Ile Phe Ser Lys Asn Cys Asn Asn Ser Phe Ala Glu Cys Ser Ser Gln Gly
                        140
```

# FIG. 1-2

```
                      740                                         760
CAT GAG AGG CAG TCC GAG GGA TCC TCC AGC CCG CAG CTC CAG GAG TCT
His Glu Arg Gln Ser Glu Gly Ser Ser Ser Pro Gln Leu Gln Glu Ser
                                    160
      780                             800                               820
GTC TTC CAC CTG CTG GTG CCC AGT GTC ATC CTG GTC TTG CTG GCC GTC GGA
Val Phe His Leu Leu Val Pro Ser Val Ile Leu Val Leu Leu Ala Val Gly
                                                        180
                         840                                  860
GGC CTC TTG TTC TAC AGG TGG AGG CGG CGG AGC CAT CAA GAG CCT CAG
Gly Leu Leu Phe Tyr Arg Trp Arg Arg Arg Ser His Gln Glu Pro Gln

      880                             900                               920
AGA GCG GAT TCT CCC TTG GAG CAA CCA GAG GGC AGC CCC CTG ACT CAG GAT
Arg Ala Asp Ser Pro Leu Glu Gln Pro Glu Gly Ser Pro Leu Thr Gln Asp
200
                          940                      960
GAC AGA CAG GTG GAA CTG CCA GTG TAG AGGGAATTCTA  AGACCCCTCA
Asp Arg Gln Val Glu Leu Pro Val
            220
     980                       1000                        1020
CCATCCTGGA  CACACTCGTT  TGTCAATGTC  CCTCTGAAAA  TGTGACGCCC  AGCCCCGGAC

     1040                   1060                   1080
ACAGTACTCC  AGATGTTGTC  TGACCAGCTC  AGAGAGAGTA  CAGTGGGACT  GTTACCTTCC

     1100                   1120                   1140
TTGATATGGA  CAGTATTCTT  CTATTTGTGC  AGATTAAGAT  TGCATTAGTT  TTTTTCTTAA

     1160                   1180                   1200
CAACTGCATC  ATACTGTTGT  CATATGTTGA  GCCTGTGGTC  TATTAAAACC  CCTAGTTCCA

     1220                   1240                   1260
TTTCCATAA  ACTTCTGTCA  AGCCAGACCA  TCTCTACCCT  GTACTTGGAC  AACTTAACTT
       ▲
     1280                   1300                   1320
TTTTAACCAA  AGTGCAGTTT  ATGTTCACCT  TTGTTAAAGC  CACCTTGTGG  TTTCTGCCCA

     1340                   1360                   1380
TCACCTGAAC  CTACTGAAGT  TGTGTGAAAT  CCTAATTCTG  TCATCTCCGT  AGCCCTCCCA

     1400                   1420                   1440
GTTGTGCCTC  CTGCACATTG  ATGAGTGCCT  GCTGTTGTCT  TTGCCCATGT  TGTTGATGTA

     1460                   1480                   1500
GCTGTGACCC  TATTGTTCCT  CACCCCTGCC  CCCCGCCAAC  CCCAGCTGGC  CCACCTCTTC

     1520                   1540                   1560
CCCCTCCCAC  CCAAGCCCAC  AGCCAGCCCA  TCAGGAAGCC  TTCCTGGCTT  CTCCACAACC

     1580                   1600                   1620
TTCTGACTGC  TCTTTTCAGT  CATGCCCCTC  CTGCTCTTTT  GTATTTGGCT  AATAGTATAT

     1640
CAATTTGCAC  TT
```

# FIG. 2-1

```
    CCCTGCTGTTGTTGGTCTGTCTCCTGGCGAGCAGGAGTATCACC   44
-14 LeuLeuLeuLeuValCysLeuLeuAlaSerArgSerIleThr

    GAGGAGGTGTCGGAGTACTGTAGCCACATGATTGGGAGTGGACACCTGCAGTCTCTGCAG 104
  1 GluGluValSerGluTyrCysSerHisMETIleGlySerGlyHisLeuGlnSerLeuGln

    CGGCTGATTGACAGTCAGATGGAGACCTCGTGCCAAATTACATTTGAGTTTGTAGACCAG 164
 21 ArgLeuIleAspSerGlnMETGluThrSerCysGlnIleThrPheGluPheValAspGln

    GAACAGTTGAAAGATCCAGTGTGCTACCTTAAGAAGGCATTTCTCCTGGTACAAGACATA 224
 41 GluGlnLeuLysAspProValCysTyrLeuLysLysAlaPheLeuLeuValGlnAspIle

    ATGGAGGACACCATGCGCTTCAGAGATAACACCCCCAATGCCATCGCCATTGTGCAGCTG 284
 61 METGluAspThrMETArgPheArgAspAsnThrProAsnAlaIleAlaIleValGlnLeu

    CAGGAACTCTCTTTGAGGCTGAAGAGCTGCTTCACCAAGGATTATGAAGAGCATGACAAG 344
 81 GlnGluLeuSerLeuArgLeuLysSerCysPheThrLysAspTyrGluGluHisAspLys

    GCCTGCGTCCGAACTTTCTATGAGACACCTCTCCAGTTGCTGGAGAAGGTCAAGAATGTC 404
101 AlaCysValArgThrPheTyrGluThrProLeuGlnLeuLeuGluLysValLysAsnVal

    TTTAATGAAACAAAGAATCTCCTTGACAAGGACTGGAATATTTTCAGCAAGAACTGCAAC 464
121 PheAsnGluThrLysAsnLeuLeuAspLysAspTrpAsnIlePheSerLysAsnCysAsn

    AACAGCTTTGCTGAATGCTCCAGCCAAGATGTGGTGACCAAGCCTGATTGCAACTGCCTG 524
141 AsnSerPheAlaGluCysSerSerGlnAspValValThrLysProAspCysAsnCysLeu

    TACCCCAAAGCCATCCCTAGCAGTGACCCGGCCTCTGTCTCCCCTCATCAGCCCCTCGCC 584
161 TyrProLysAlaIleProSerSerAspProAlaSerValSerProHisGlnProLeuAla

    CCCTCCATGGCCCCTGTGGCTGGCTTGACCTGGGAGGACTCTGAGGGAACTGAGGGCAGC 644
181 ProSerMETAlaProValAlaGlyLeuThrTrpGluAspSerGluGlyThrGluGlySer

    TCCCTCTTGCCTGGTGAGCAGCCCCTGCACACAGTGGATCCAGGCAGTGCCAAGCAGCGG 704
201 SerLeuLeuProGlyGluGlnProLeuHisThrValAspProGlySerAlaLysGlnArg

    CCACCCAGGAGCACCTGCCAGAGCTTTGAGCCGCCAGAGACCCCAGTTGTCAAGGACAGC 764
221 ProProArgSerThrCysGlnSerPheGluProProGluThrProValValLysAspSer

    ACCATCGGTGGCTCACCACAGCCTCGCCCCTCTGTCGGGGCCTTCAACCCCGGGATGGAG 824
241 ThrIleGlyGlySerProGlnProArgProSerValGlyAlaPheAsnProGlyMETGlu

    GATATTCTTGACTCTGCAATGGGCACTAATTGGGTCCCAGAAGAAGCCTCTGGAGAGGCC 884
261 AspIleLeuAspSerAlaMETGlyThrAsnTrpValProGluGluAlaSerGlyGluAla

    AGTGAGATTCCCGTACCCCAAGGGACAGAGCTTTCCCCCTCCAGGCCAGGAGGGGGCAGC 944
281 SerGluIleProValProGlnGlyThrGluLeuSerProSerArgProGlyGlyGlySer

    ATGCAGACAGAGCCCGCCAGACCCAGCAACTTCCTCTCAGCATCTTCTCCACTCCCTGCA 1004
301 METGlnThrGluProAlaArgProSerAsnPheLeuSerAlaSerSerProLeuProAla

    TCAGCAAAGGGCCAACAGCCGGCAGATGTAACTGGTACAGCCTTGCCCAGGGTGGGCCCC 1064
321 SerAlaLysGlyGlnGlnProAlaAspValThrGlyThrAlaLeuProArgValGlyPro

    GTGAGGCCCACTGGCCAGGACTGGAATCACACCCCCCAGAAGACAGACCATCCATCTGCC 1124
341 ValArgProThrGlyGlnAspTrpAsnHisThrProGlnLysThrAspHisProSerAla

    CTGCTCAGAGACCCCCCGGAGCCAGGCTCTCCCAGGATCTCATCACTGCGCCCCCAGGGC 1184
361 LeuLeuArgAspProProGluProGlySerProArgIleSerSerLeuArgProGlnGly
```

CTCAGCAACCCCTCCACCCTCTCTGCTCAGCCACAGCTTTCCAGAAGCCACTCCTCGGGC 1244

381 LeuSerAsnProSerThrLeuSerAlaGlnProGlnLeuSerArgSerHisSerSerGly

AGCGTGCTGCCCCTTGGGGAGCTGGAGGGCAGGAGGAGCACCAGGGATCGGAGGAGCCCC 1304

401 SerValLeuProLeuGlyGluLeuGluGlyArgArgSerThrArgAspArgArgSerPro

GCAGAGCCAGAAGGAGGACCAGCAAGTGAAGGGGCAGCCAGGCCCCTGCCCCGTTTTAAC 1364

421 AlaGluProGluGlyGlyProAlaSerGluGlyAlaAlaArgProLeuProArgPheAsn

TCCGTTCCTTTGACTGACACAGGCCATGAGAGGCAGTCCGAGGGATCCTCCAGCCCGCAG 1424

441 SerValProLeuThrAspThrGlyHisGluArgGlnSerGluGlySerSerSerProGln

CTCCAGGAGTCTGTCTTCCACCTGCTGGTGCCCAGTGTCATCCTGGTCTTGCTGGCCGTC 1484

461 LeuGlnGluSerValPheHisLeuLeuValProSerValIleLeuValLeuLeuAlaVal

GGAGGCCTCTTGTTCTACAGGTGGAGGCGGCGGAGCCATCAAGAGCCTCAGAGAGCGGAT 1544

481 GlyGlyLeuLeuPheTyrArgTrpArgArgArgSerHisGlnGluProGlnArgAlaAsp

TCTCCCTTGGAGCAACCAGAGGGCAGCCCCCTGACTCAGGATGACAGACAGGTGGAACTG 1604

501 SerProLeuGluGlnProGluGlySerProLeuThrGlnAspAspArgGlnValGluLeu

CCAGTGTAGAGGGAATTCTAAGACCCCTCACCATCCTGGACACACTCGTTTGTCAATGTC 1664

521 ProVal...

CCTCTGAAAATGTGACGCCCAGCCCCGGACACAGTACTCCAGATGTTGTCTGACCAGCTC 1724

AGAGAGAGTACAGTGGGACTGTTACCTTCCTTGATATGGACAGTATTCTTCTATTTGTGC 1784

AGATTAAGATTGCATTAGTTTTTTTCTTAACAACTGCATCATACTGTTGTCATATGTTGA 1844

GCCTGTGGTCTATTAAAACCCCTAGTTCCATTTCCCATAAACTTCTGTCAAGCCAGACCA 1904

TCTCTACCCTGTACTTGGACAACTTAACTTTTTTAACCAAAGTGCAGTTTATGTTCACCT 1964

TTGTTAAAGCCACCTTGTGGTTTCTGCCCATCACCTGAACCTACTGAAGTTGTGTGAAAT 2024

CCTAATTCTGTCATCTCCGTAGCCCTCCCAGTTGTGCCTCCTGCACATTGATGAGTGCCT 2084

GCTGTTGTCTTTGCCCATGTTGTTGATGTAGCTGTGACCCTATTGTTCCTCACCCCTGCC 2144

CCCCGCCAACCCCAGCTGGCCCACCTCTTCCCCCTCCCACCCAAGCCCACAGCCAGCCCA 2204

TCAGGAAGCCTTCCTGGCTTCTCCACAACCTTCTGACTGCTCTTTTCAGTCATGCCCCTC 2264

CTGCTCTTTTGTATTTGGCTAATAGTATATCAATTTGC

FIG. 2-2

# FIG. 3

HUMAN CSF-I GENOMIC STRUCTURE

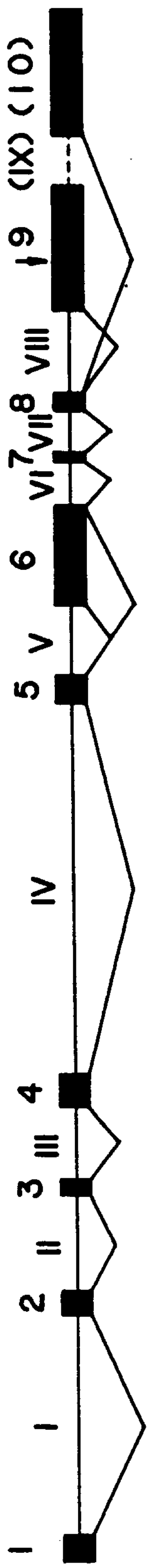

FIG. 4

RP-HPLC ANALYSIS OF SCSF/C▽158 CSF-1

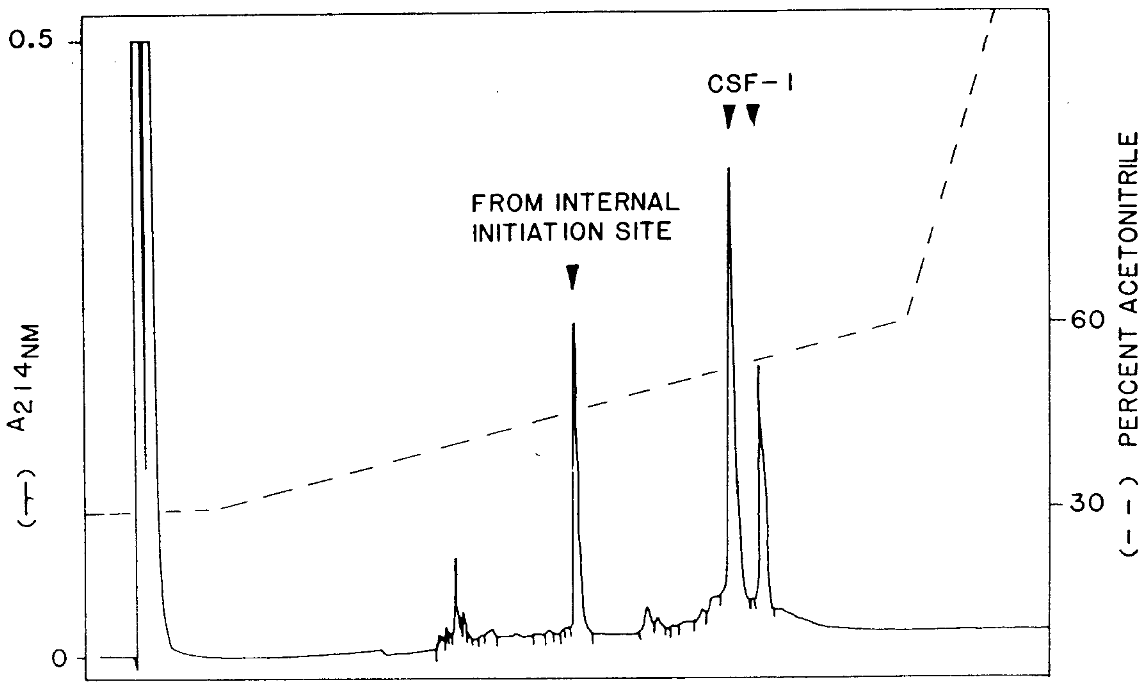


FIG. 5

RP-HPLC ANALYSIS OF $ASP_{59}$-SCSF/C▽150 CSF-1

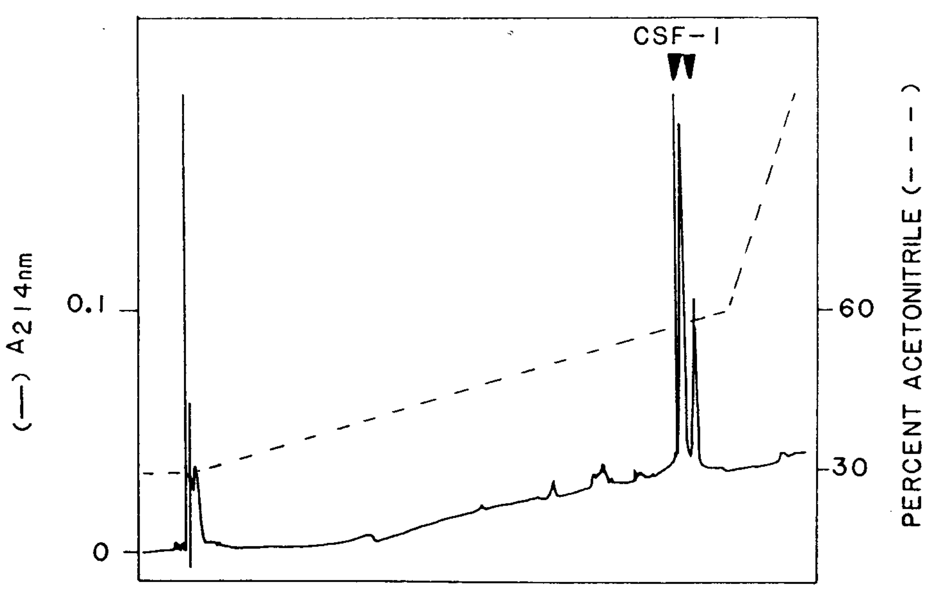

# FIG. 6

RP-HPLC ANALYSIS OF ASP59-SCSF/N∇3C∇150 CSF-1